# EUROPEAN PATENT SPECIFICATION

(11) **EP 1 543 168 B1**
(45) Date of publication and mention of the grant of the patent: **19.05.2010**
(21) Application number: 03785637.4
(22) Date of filing: 26.09.2003
(51) Int. Cl.: C12Q 1/70

(54) **METHOD FOR ASSAYING REPLICATION OF HBV AND TESTING SUSCEPTIBILITY TO DRUGS**
METHODE ZUR DARSTELLUNG DER REPLIKATION VON HBV UND ZUM TESTEN DER SENSIBILITÄT VON WIRKSTOFFEN
PROCEDE D'ANALYSE DE LA REPLICATION DU HBV ET D'ESSAI DE LA SENSIBILITE AUX MEDICAMENTS

(30) Priority: 27.09.2002 EP 02356188
(43) Date of publication of application: 22.06.2005
(73) Proprietor: INSTITUT NATIONAL DE LA SANTE ET DE LA RECHERCHE MEDICALE (INSERM), 75013 Paris (FR)
(72) Inventor: DURANTEL, David, F-69740 Genas (FR); DURANTEL, Sandra, F-69740 Genas (FR); TREPO, Christian, F-69500 Bron (FR); ZOULIM, Fabien, F-69570 Dardilly (FR)
(74) Representative: Colombet, Alain André
(86) International application number: PCT/EP2003/012398
(87) International publication number: WO 2004/029301

(56) References cited:
- SCHORIES MARCUS ET AL: "Isolation, characterization and biological significance of hepatitis B virus mutants from serum of a patient with immunologically negative HBV infection." JOURNAL OF HEPATOLOGY, vol. 33, no. 5, November 2000 (2000-11), pages 799-811, XP002246280 ISSN: 0168-8278
- ULRICH P P ET AL: "A PRECORE-DEFECTIVE MUTANT OF HEPATITIS B VIRUS ASSOCIATED WITH E ANTIGEN-NEGATIVE CHRONIC LIVER DISEASE" JOURNAL OF MEDICAL VIROLOGY, vol. 32, no. 2, 1990, pages 109-118, XP008019147 ISSN: 0146-6615
- GUNTHER STEPHAN ET AL: "Amplification of full-length hepatitis B virus genomes from samples from patients with low levels of viremia: Frequency and functional consequences of PCR-introduced mutations." JOURNAL OF CLINICAL MICROBIOLOGY, vol. 36, no. 2, February 1998 (1998-02), pages 531-538, XP002246281 ISSN: 0095-1137
- DELANEY W E 4TH ET AL: "Use of the hepatitis B virus recombinant baculovirus-HepG2 system to study the effects of (-)-beta-2',3'-dideoxy-3'-thiacytidine on replication of hepatitis B virus and accumulation of covalently closed circular DNA." ANTIMICROBIAL AGENTS AND CHEMOTHERAPY. UNITED STATES AUG 1999, vol. 43, no. 8, August 1999 (1999-08), pages 2017-2026, XP002246282 ISSN: 0066-4804
- DATABASE EBI [Online] 6 October 1999 (1999-10-06), FRANK, B.L., ET AL.: "Sequence 5 from patent US 5856459" XP002246286 accession no. EMBL Database accession no. AR027807 & US 5 856 459 A (HYBRIDON, INC.) 5 January 1999 (1999-01-05)
- DATABASE GENBANK [Online] 23 March 2001 (2001-03-23), SUGAUCHI, F. ET AL.: "Hepatitis B virus DNA, complete genome, serotype: ayw" XP002246287 retrieved from GI:13365548 accession no. NCBI Database accession no. AB048704 cited in the application
- KRUINING JOHANNES ET AL: "Antiviral agents in hepatitis B virus transfected cell lines: Inhibitory and cytotoxic effect related to time of treatment." JOURNAL OF HEPATOLOGY, vol. 22, no. 3, 1995, pages 263-267, XP002246283 ISSN: 0168-8278
- DELMAS JULIEN ET AL: "Inhibitory effect of adefovir on viral DNA synthesis and covalently closed circular DNA formation in duck hepatitis B virus-infected hepatocytes in vivo and in vitro." ANTIMICROBIAL AGENTS AND CHEMOTHERAPY. UNITED STATES FEB 2002, vol. 46, no. 2, February 2002 (2002-02), pages 425-433, XP002246284 ISSN: 0066-4804
- BECK J ET AL: "Reconstitution of a functional duck hepatitis B virus replication initiation complex from separate reverse transcriptase domains expressed in Escherichia coli." JOURNAL OF VIROLOGY. UNITED STATES AUG 2001, vol. 75, no. 16, August 2001 (2001-08), pages 7410-7419, XP002246285 ISSN: 0022-538X
- DELANEY ET AL.: "Hepatitis B Virus Replication in Human HepG2 Cells Mediated by Hepatitis B Virus Recombinant Baculovirus" HEPATOLOGY, vol. 28, no. 4, October 1998 (1998-10), pages 1134-1146, XP008019212

## Description

The present invention relates to the cloning of replication-competent HBV DNA genomes, to methods for measuring the replication capacity of HBV, e.g. HBV present in a biological sample, possibly in the presence of a pharmaceutical product, in particular an antiviral agent. The invention relates in particular to phenotypic assays allowing to test the susceptibility of HBV to drugs or to screen drugs on HBV.

More particularly, the invention concerns such methods to determine the susceptibility of the viral strains present in a patient to the available drugs and therefore tailor antiviral therapy to the virological situation.

The invention also relates to primers, primers pairs and kits used in amplification, as well as to vectors comprising replication-competent HBV DNA genomes and cells, e.g. cell lines transfected with such vectors.

Despite the development of an efficient vaccine, chronic Hepatitis B virus (HBV) infection is still a major public health problem worldwide. Indeed, more than 400 million people are chronic carriers of the virus and are at high risk of developing cirrhosis and hepatocellular carcinoma.

HBV is a DNA virus that replicates its genome via a reverse transcriptase phase. The spontaneous error rate of the viral reverse transcriptase leads to the heterogeneity of HBV genome. Illustrating the latter point, eight genotypes (A to H) have been identified, and HBV mutants which are selected either during the natural course of the infection or during therapy, have been described.

Recently, major progress has been made in the field of antiviral therapy of chronic hepatitis B, with the development of nucleoside analogues that inhibit the viral polymerase. Hence, lamivudine has been approved two years ago and is now largely used in clinical practice worldwide. Although it inhibits significantly viral replication, the emergence of drug resistant mutants harbouring mutations in conserved domains of the viral polymerase is observed with a prevalence in patient increasing from 15 at year one to 75 % at year four of therapy. As these mutants may represent a new threat for human health, new antiviral agents were needed to combat them. Thus, other inhibitors, such as adefovir dipivoxil, entecavir, emtricitabine, clevudine, and telbivudine, have been recently studied for their anti-HBV properties in experimental models and are currently evaluated in clinical trials. However, *in vitro* studies have also shown that lamivudine resistant strains may be cross-resistant to some of these drugs (e.g. emtricitabine), while sensitive to other. This observation warrants a close monitoring of viral population *in vivo* and emphasises the development of drug resistance assay (or phenotypic assay).

Therefore, it will be important in the future, in the clinical setting, to know in each patient the susceptibility of the viral strains to the available drugs and therefore tailor antiviral therapy to the virological situation.

To date, no efficient phenotypic drug susceptibility assay has been developed for HBV. With the current and future development of new anti-HBV molecules, phenotypic drug susceptibility testing should become an important issue for the management of patient infected with resistant HBV isolates.

The mechanism of HBV entry into cells has not been completely elucidated. After uncoating, the HBV DNA is brought into the nucleus of the host cell, where it is repaired to the covalently closed circular form (cccDNA). Once recircularized, enhancer and promoter activities start producing the various HBV RNA transcripts. There are sub-genomic transcripts which serve as mRNA for the expression of the X and the surface proteins. The larger genomic transcripts are longer than one genome in length and serve to produce e, core and polymerase proteins. And a particular genomic transcript, lacking the ATG start codon for the e protein, is designated the pregenomic RNA (pgRNA). This pgRNA, which is the template for the synthesis of HBV DNA by reverse transcription, is longer than one genome in length (i.e. about 1.1 genome unit) and therefore contains a terminally-redundant RNA strand. The redundant sequence comprises about 200 nucleotides, including direct repeat 1 (DR1) as well as the stem-loop. See for more details G-H Wang et al., Cell 1992, 71: 663-670, G-H Wang et al., J. Virol. 1993, 67: 6507-6512).

When studying HBV mutants *in vitro,* plasmidic vectors containing the genomic information required for the initiation of HBV replication in cells after transfection are used; such plasmids are replication competent. These plasmids contain from 1.1 to 2 HBV genomes. Classic methods to study mutants rely on site directed mutagenesis of well established replication competent HBV constructs (laboratory strains only, not clinical) or on the introduction of HBV genome fragments (i.e. "cassette exchange") from clinical isolates in replication-competent HBV constructs (idem). PCR-mediated transfer of HBV genome cassettes or on site directed mutagenesis within a well established replication-competent laboratory strain allowed for instance to confirm that some mutants selected *in vivo* in patients during antiviral therapy were indeed conferring resistance to lamivudine (Seigneres B., et al., Hepatology 2002, 36: 710-722; Pichoud C. et al., Hepatology 1999, 29: 230-237; Allen M.I. et al., Hepatology 1998, 27: 1670-1677; Bock C.T. et al., Gastroenterology 2002, 122: 264-273). However these methods do not allow to studying the whole HBV genetic information from the clinical isolates and may therefore exclude some important mutations located in other parts of the genome or result in mosaic HBV genomes.

Günther et al (J. Virol. 1995, 69, 9: 5437-5444) have first described a method for PCR amplification of full-length HBV genomes (i.e. one genome unit) that was meant to facilitate the analysis of natural HBV isolates. The full-length amplicon was either directly used for transfection experiment of hepatoma cell line or cloned prior to its use. Both approaches involved the utilization of the Sap-I restriction enzyme (From New England Biolabs), which is a quite expensive and inefficient restriction enzyme. The tranfected full-length genome is circularized in the nucleus of cells by host enzymes (which remain to be identified) to give rise to covalently closed circular HBV DNA, which is the natural template for transcription of HBV genes. In this case the endogenous HBV promoter is used for the transcription. The same method is used in S. Günther et al., J. Clin. Microb 1998, 36, 2 : 531-538). The process of host mediated circularization of linear HBV DNA is highly inefficient and represent therefore a major limitation for the analysis of all HBV mutants. Due to the low level of HBV circular template generated a low level of HBV DNA synthesis (replication) is obtained. This low level of HBV DNA synthesis hampers the analysis of viral replication and drug susceptibility testing. Other limitations of the Günther's method are the high amount of amplification products that is necessary for cell transfection, and the absence of "an easy to use" continuous source of material for cell transfections and phenotypic studies. Although interesting for fundamental research, this method does not seem appropriate in the context of a standardized phenotypic assay which requires solid replication level and production of easily detectable amount of viral particles in the cell culture medium.

M. Schories et al. (J. of Hepatology 2000, 33, 799-811), describe the molecular characterization of an HBV mutant isolated from a patient via 2 PCR amplification of overlapping regions. After identification of an interesting mutation, a sub-cloning strategy (referred as "cassette exchange" above) was used to insert the mutation in the background of a laboratory HBV strain. The clones obtained contain two HBV genomes.

P.P. Ulrich et al., J. of Medical Virology 1990, 32 : 109-118, describe the molecular characterization of an HBV mutant isolated from a patient via amplification of the circulating DNA. Mutations were detected by sequencing. For biologic evaluation of these mutations on HBV replication and expression of HBeAg, *in vitro,* HepG2 cells were transfected with the cloned, re-circularised mutant HBV DNA. This document teaches that a complete (one unit) HBV genome can be amplified by PCR and is replication competent *in vitro.* W.E. Delaney et al. describes construction of a baculovirus vector containing a 1.3 HBV unit genome allowing infection of eucaryote cells and production of 1.3 HBV genomes in these cells.

Most of works described in these prior publications are based on mutagenesis or cassette exchange strategies (cloning or sub-cloning of fragment). In all cases the vectors constructed are using the endogenous promoter and cloning (or subcloning) methods do not comprise means allowing the rapid and standardized cloning of HBV DNA from samples. Therefore, they can not be used in clinical survey or in drug susceptibility assays.

J. Kruining et al., J. Hepatol. 1995, 22 : 263-267 describes cell lines stably transfected with HBV. These cells lines are used to test drugs but do not concern testing drugs with respect to clinical HBV mutants.

There is thus still a need for an efficient method allowing to evaluate the replication capacity of HBV and to test the susceptibility of HBV isolates to drugs and/or to test the antiviral activity of drugs on HBV, in particular in clinical survey.

An objective of the invention is to propose a rapid and reproducible cloning of replication-competent HBV DNA genomes from patient samples.

Another objective of the invention is to propose a rapid and reproducible phenotypic assay method allowing to test the susceptibility of HBV isolates with respect to drugs. In particular, this object aims to determine the susceptibility of the viral strains present in a patient to the available drugs and therefore tailor antiviral therapy to the virological situation.

Another objective of the invention is to propose a method allowing to rapidly produce *in vitro* HBV replication competent clones useful for phenotypic analysis or for functional analysis.

Still another objective of the invention is to propose a rapid and reproducible assay method allowing to test the antiviral activity of current and future drugs to particular HBV or to HBV variants or to HBV field isolates.

Still another objective is to propose a method allowing to study the replication capacity of wild-types and mutants of HBV

Still another objective is to allow the production of cell lines constitutively producing HBV particles.

The present invention is based on the cloning into a vector of HBV DNA covering more-than-full-length (i.e. cloning of about 1.1 genome unit) HBV genome, such that a pgRNA can be synthesized from this DNA post-cell-transfection. The pgRNA synthesis in the host cells initiates the HBV intracellular cycle with viral gene expression, including synthesis of intracellular replicative intermediates, which comprise single stranded DNA, double stranded linear DNA, relaxed circular DNA and the minor species of replicative intermediates. The invention allows the rapid cloning of replication-competent HBV DNA genomes. Then, it is possible to evaluate the level of HBV DNA synthesis in the cells, e.g. by Southern blot, or of RNAs synthesis, e.g. by Northern blot, or of viral protein expression, e.g. by Western blot, ELISA or RIA, in the presence or in the absence of antiviral agent to be evaluated. It is also possible to analyse the level of virus production, in particular by measuring the level of virion DNA or of Dane particles in the extra-cellular medium or supernatant, using Southern blot or real time PCR method or similar, in the presence or in the absence of antiviral agent to be evaluated.

A first object of the invention is thus a method for measuring the replication capacity of HBV, e.g. HBV present in a biological sample, possibly in the presence of a pharmaceutical product, in particular an antiviral agent, the method comprising:
(a) possibly extracting nucleic acids contained in the biological sample;
(b) PCR amplifying HBV nucleic acids using at least two primer pairs selected so as to obtain at least two amplified HBV genomic fragments representing more-than-full-length HBV genome and which upon assembly represent a linear continuous DNA sequence transcriptable in pgRNA ;
(c) cloning the fragments obtained under (b) into a vector under the control of a promoter, preferably a heterologous promoter, so producing a vector containing a linear continuous DNA sequence (about 1.1 genome unit in length) transcriptable in pgRNA under control of said promoter;
(d) transfecting or transducing susceptible cells with the vector;
(e) culturing the transfected or transduced cells in conditions allowing synthesis of HBV pgRNA from the cloned HBV DNA;
(f) possibly treating the cultured cells with the pharmaceutical product, in particular antiviral agent; and
(g) determining the replication capacity of the HBV, possibly incidence of the pharmaceutical product, in particular antiviral agent, on viral gene expression and/or viral replication.

The biological sample may be derived from a patient. It can be a sample of blood or serum, but can be any body fluid or else a liver biopsy sample, or any preparation issued therefrom. The biological sample may also be derived from a viral culture. It can be a cell culture supernatant. Generally speaking a biological sample can be any type of sample containing one or several HBV clones.

Step (a) provides the extraction of DNA from the biological sample. This can be made by any usual method. These methods may comprise a chromatography column-based purification, in particular affinity chromatography, and/or differential precipitation of proteins and nucleic acids. Use may be made of commercial purification kits, as those used in the examples.

The cloning of the HBV DNA covering more-than-full-length HBV genome is done by amplifying (step (b)) at least two, preferably two, HBV genomic fragments, then subjecting the amplicons with suitable restriction enzymes to ligate the whole insert in the vector (step (c)).

Each HBV genomic fragment is amplified in step (b) using suitable primer pairs.

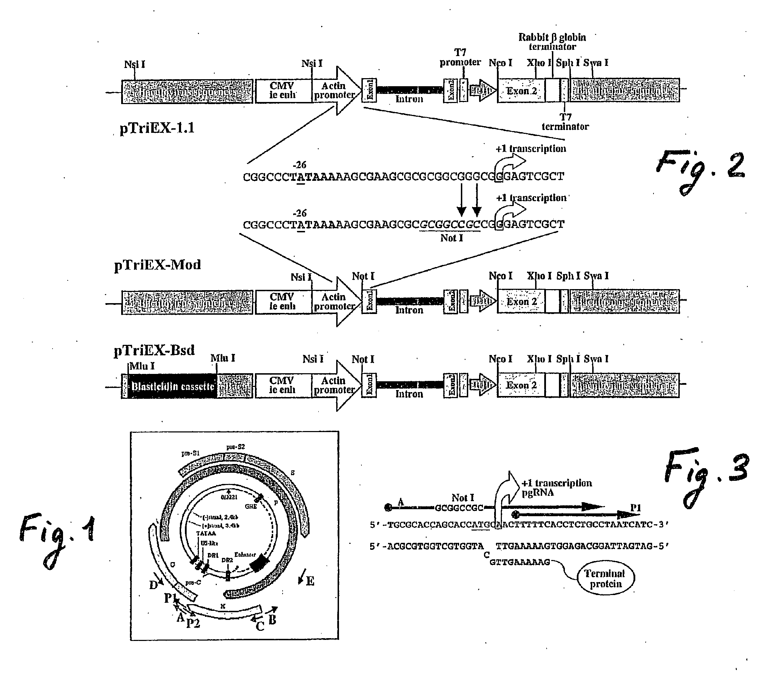

The HBV are classified into the genetic groups A to H based on divergence of the complete nucleotide sequence. Such sequences are available in articles and in GenBank for each genotype. In the present specification, the numbering of nucleotides is by reference to the whole HBV genomic DNA sequence published in H. Norder et al. (Virology 1994, 198, 489-503, incorporated herein by reference; Figure 1 of this article presents an alignment of the genome of various HBV clones representing genotypes C, E and F, with the sequence of clone pHBV-3200 which is 3221 nucleotides long) or the one of a genotype C HBV published in GenBank under access number AB048704 (incorporated herein by reference). The length of the genome of the various HBV is variable. That is to say that numbering of nucleotides should only be considered as illustrative embodiments.

As it will be described in more details *infra,* according to a very advantageous characteristic of the invention, use is made of primers that are complementary to a well-conserved region inside HBV genotypes. These primers are thus able to hybridise to the various HBV present in a sample, representing the so-called quasi-species. The method allows one to clone and amplify the quasi-species, and then to apply the tests according to the invention not only with respect to one HBV isolate, but with respect to the HBV population infecting a patient.

In a first embodiment, the aim is to clone a more-than-full-length (i.e. about 1.1 genome unit) HBV genome under the control of an heterologous promoter. The vector is made containing a heterologous promoter operatively linked to the HBV sequence so that a pgRNA may be synthesized under the control of that promoter. And the primer pairs for PCR amplifications are made so as to allows one to obtain this continuous HBV DNA sequence transcriptable in pgRNA, which DNA sequence represents more than one HBV unit genome (i.e. about 1.1 genome unit; so-called more-than-full length). Advantageously, the heterologous promoter is strong which means that transcription level is higher than with the endogenous promoter.

In this embodiment, by definition, a more-than-full-length HBV genome is a continuous sequence comprising, or consisting essentially of, HBV nucleotides (from 5' to 3'):
- an about 1-unit genome starting in 5' from and including the nucleotide representing the +1 of transcription (in general nucleotide A, position 1818 in H. Norder et al.; in some cases however, the +1 of transcription has revealed to be the nucleotide in 5' of said A, i.e. C, or in 3', i.e. A) to the first nucleotide in 5' of the ATG of the pre-C gene (nucleotide 1813 in H. Norder et al.), plus
- a sub-genomic fragment starting from and including the A of the ATG of the pre-C gene (nucleotide 1814 in H. Norder et al.) and extending to and including the polyA attachment site (nucleotide 1960 in H. Norder et al.), thus comprising the polyA sequence TATAAA, nucleotides 1916-1920 in H. Norder et al.). These fragments are overlapping if one refers to the circular genome. The whole sequence corresponds to at least about 1.1 genome unit (it is by definition a more-than-full-length genome) and is able to produce a pgRNA.

If one refers to H. Norder at al. or in GenBank AB048704, the more-than-full-length HBV genome comprises from 5' to 3' nucleotides 1818 to 1813 and 1814 to 1960. The number of nucleotides may be variable depending on the HBV. For instance, some HBV lack several nucleotides at positions 2806 to 2611 and 2884 to 2886 (Norder et al., Figure 1). It is however evident that this teaching extends itself to any HBV. In one embodiment, the more-than-full-length HBV genome comprises from 5' to 3' nucleotides 1818 to 1813 and 1814 to 1960 of an HBV genomic sequence aligned with the one disclosed in H. Norder et al. or in GenBank AB048704 (alignment means that spaces are introduced for the missing nucleotides, as was done for instance in Norder et al., or that possible supplemental nucleotides are not considered). In a particular embodiment, the more-than-full-length HBV genome comprises nucleotides 1816 to 1813 and 1814 to 2016 of an HBV genomic sequence aligned with the sequence as mentioned above. Genomic map of an HBV clone is easily available after sequence alignment so that the one skilled in the art may easily determine with precision for every HBV clone any HBV genomic region and in particular those used in the invention, such as the +1 of transcription, the pre-C gene and its ATG, the polyA attachment site, so that the so-called more-than-full-length HBV genome can be determined.

Also, the more-than-full-length HBV genome can be longer and for instance can comprise further nucleotides situated in 5' of the said +1 of transcription. In that event however, it is necessary that the longer sequence does not comprise in 5' a functional ATG for the pre-C gene (A and/or T and/or G mutated or deleted) and preferably do not contain the endogenous HBV promoter which physiologically drives the expression of pgRNA (located between position 1744 and 1795). Also, it can be longer at the 3' end. In practice, one may bear in mind that: the location of the primer determines the length of the sequence; it is preferred to choose a primer which is complementary to a well-conserved region inside HBV genotypes and as close as possible of the polyA attachment site; and the sensibility of the PCR is better with smaller amplicons.

In this first embodiment, amplification is preferably made of two fragments whose DNA sequence comprises HBV nucleotides (from 5' to 3') :
- the first fragment comprises a HBV DNA sequence comprising at its 5' end the nucleotide representing the +1 of transcription, and
- the second fragment comprises a HBV DNA sequence comprising at its 3' end the polyA attachment site
the 3' end of the first fragment and the 5' end of the second fragment being overlapping and preferably comprising a restriction site in the overlapping part, so that upon restriction treatment both fragments can be ligated to give rise to the more-than-full-length HBV genome.

Two primers pairs are designed to amplify such first and second amplicons, respectively.

The primers disclosed herein have been designed after multiple sequence alignment with more than 200 whole HBV genomes, and in the end checked with respect to a total of about 500 whole HBV genomes. As it is the case with the herein disclosed primers, primers useful in the invention are better designed from well-conserved regions, in order to allows one to apply the invention regardless of the genotypes and main natural mutations of the field HBV. In order to allow representing most if not all HBV genomes, an embodiment provides for the use of a mixture of different primers which differ only by the variations necessary to represent all possibilities. For instance, if the HBV genomes can be classified into two families regarding a specific genomic region targeted by the primer, the primer designed to be complementary to this region may be a mixture of two primers, representing each one of these groups.

In this first embodiment, a preferable first primer pair comprises a forward primer A which is partially complementary to the 5' part of the pre-C gene (which codes for protein e), including the nucleotide representing the +1 of transcription (as defined above), but does not contain the ATG of the pre-C gene.

Suitable A primers are by way of example:
SEQ ID NO: 1
   5'TGCGCACCGCGGCCGCGCAACTTTTTCACCTCTGCC 3'
   This primer comprises a *Not*I site (once underlined) downstream of the G of the start codon of pre-C, and hybridise on nucleotides 1816 to 1835 (H. Norder et al., and GenBank AB048704).
SEQ ID NO: 2
   5' TGCGCACCAGGGCGCGCCAACTTTTTCACCTCTGCC 3'
   This primer comprises a *Asc*I site (once underlined).
SEQ ID NO: 3
   5' TGCGCACCCCTGCAGGGCAACTTTTTCACCTCTGCC 3'
   This primer comprises a *Sse*8387 I site (once underlined).
SEQ ID NO: 4
   5' TGCGCACCAGGTTTAAACAACTTTTTCACCTCTGCC 3'
   This primer comprises a *Pme*I site (once underlined).
SEQ ID NO: 5
   5' TGCGCACCAGCGGCCGCCAACTTTTTCACCTCTGCC 3'
   This primer comprises a *Not*I site (once underlined).
SEQ ID NO: 6
   5' TGCGCACCACCTGCAGGCAACTTTTTCACCTCTGCC 3'
   This primer comprises a *Sse*8387 I site (once underlined).
SEQ ID NO: 7
   5' TGCGCACCAGGTTTAAACAACTTTTTCACCTCTGCC 3'
   This primer comprises a *Pme*I site (once underlined).
SEQ ID NO: 8
   5' TGCGCACCAGGCGCGCCCAACTTTTTCACCTCTGCC 3'
   This primer comprises a *Asc*I site (once underlined).
SEQ ID NO: 9
   5' TGCGCACGGCGCGCCTGCAACTTTTTCACCTCTGCC 3'
   This primer comprises a *Asc*I site (once underlined).
SEQ ID NO: 10
   5' TGCGCACCCTGCAGGTGCAACTTTTTCACCTCTGCC 3'
   This primer comprises a *Sse*8387 I site (once underlined).
SEQ ID NO: 11
   5' TGCGCACCGCGGCCGCGCAACTTTTTCACCTCTGCC 3'
   This primer comprises a *Not*I site (once underlined).
SEQ ID NO: 12
   5' TGCGCACCATTAATTAACAACTTTTTCACCTCTGCC 3'
   This primer comprises a *Pac*I site (once underlined).

In this first embodiment, it is preferred to clone HBV DNA in order to fuse the natural +1 of transcription of the HBV pgRNA to the +1 of transcription determined by the promoter contained in the vector or plasmid used for the cloning. To achieve this fusion at molecular level, the promoter may be first modified by the insertion (mutagenesis), between the TATA box (or equivalent) of the basic promoter and the +1 of transcription, of a restriction site which is preferably not present in any of the known complete HBV sequences. Several restriction enzymes that recognize a palindromic site of 8 nucleotides, such as *Not*I, *Asc*I, *Pac*I, *Pme*I, *SSe*8387l, do not cut HBV genome, and can be used for this strategy. The same restriction site is also integrated into the tail of the primer A used for the PCR. SEQ ID NO: 1 to 12 are examples of primers useful in this embodiment, comprising such restriction sites. Of course, it is evident that the one skilled in the art is able to prepare other suitable primers.

Generally speaking, polynucleotides useful as preferable primers in this embodiment comprise a nucleotide sequence corresponding to a restriction site, preferably not present in the HBV genome, situated in 5' of a sequence complementary to the 5' end of the pre-C gene, but lacking the complete ATG of this pre-C gene (e.g. A, or A and T or A and T and G are missing). As appearing on the sequences referred SEQ ID NO: 1 to 12, the restriction site can be directly linked to the +1 of transcription or separated by some nucleotides (e.g. 1-3). In the preferred embodiment, the primer comprises SEQ ID NO: 1. On the sequences, the double underlined sequence is complementary to the 5' part of the pre-C gene. Of course, the 3' end thereof may be shortened or extended to contain further complementary nucleotides. If one wants to keep the possibility of hybridising DNA from any HBV, it is preferable to not extend the primer so that it includes non-conservative regions.

Thus the cloning of the HBV DNA involves the use of a heterologous promoter constructed so as to contain a restriction site not present in HBV genomes and of a primer hybridised to the HBV DNA and comprising the same restriction site, so that fusion of the HBV DNA and the promoter may occur. Preferably, the natural +1 of transcription of the HBV pgRNA is so fused to the +1 of transcription determined by the promoter.

This first primer pair comprises preferably a reverse primer chosen to be complementary to a consensus or a substantially consensus sequence within the HBV genomes.

Suitable reverse B primers are by way of example:
B1: SEQ ID NO: 13
   5' GGCAGCACASCCTAGCAGCCATGG 3'
   This primer comprises a *Nco*l site (underlined) and are useful for amplification of genotypes A, B, D, E and G. The primer hybridizes with nucleotides 1395-1372 (H. Norder et al., and GenBank AB048704).
B2: SEQ ID NO: 14
   5' GGCAGCACASCCGAGCAGCCATGG 3'
   This primer comprises a *Nco*l site (underlined) and are useful for amplification of genotypes C and F. The primer hybridizes with nucleotides 1395-1372.

A mixture of both primers B1 and B2 may advantageously be used.

S means G or C. Thus in SEQ ID NO: 13 and 14, S can represent G or C. A mixture of both can be used. Generally, the primers will be synthesized with degeneracy, and will thus comprise both possibilities.

The second primer pair comprises preferably a forward primer chosen to be complementary to a consensus or a substantially consensus sequence within the HBV genomes; this primer is designed to be complementary to a region of HBV DNA which is in 5' with respect to that complementary with the reverse primer of the first primer pair. An example of such a forward primer is primer E described herein after and having the sequence as set forth in SEQ ID NO: 19. The best is to have both these forward and reverse primers complementary to HBV genomic regions which overlap and comprise a unique natural restriction site, e.g. the *Nco*l site at nucleotides 1372-1377.

Suitable forward primers are by way of example:
C1: SEQ ID NO: 15
   5' ACMTCSTTTCCATGGCTGCTAGG 3'
   This primer comprises a *Nco*I site (underlined) and are useful for amplification of genotypes A, B, D, E and G. The primer hybridizes with nucleotides 1363-1385 (H. Norder et al., and GenBank AB048704).
C2: SEQ ID NO: 16
   5' ACMTCSTTTCCATGGCTGCTCGG 3'
   This primer comprises a Ncol site (underlined) and are useful for amplification of genotypes C and F. The primer hybridizes with nucleotides 1363-1385.

A mixture of both primers C1 and C2 may advantageously be used.

M means A or C, and W means A or T. Thus in SEQ ID NO: 15 and 16, M can represent A or C, and W can represent A or T. A mixture can be used. Generally, the primers will be synthesized with degeneracy, and will thus comprise the various possibilities.

The second primer pair comprises preferably a suitable reverse primer chosen to be complementary to a consensus or a substantially consensus sequence within the HBV genomes; this primer is designed to be complementary to a region of the HBV genome which is in 3' with respect to that of the forward primer in the first pair of primers and more precisely in 3' of the polyA attachment site. As a result, the cloned DNA obtained after recombination between both amplified fragments or amplicons, is longer than the HBV genome and include a redundant region including the start codon ATG of the pre-C gene and the polyA attachment site. As mentioned above, it is preferred to choose a primer which is complementary to a well-conserved region inside HBV genotypes and as close as possible in 3' of the polyA attachment site.

Suitable reverse primers D are by way of example:
D1: SEQ ID NO: 17
   5' CTAAGGGCATGCGATACAGAGCWGAGGCGG 3'
   This primer comprises a *Sph*l site (underlined). The 3' part of the primer hybridizes with nucleotides 2027-1998. The C at the 5' end corresponds to nucleotide 2039.
D2: SEQ ID NO: 18
   5' CTAAGGGTCGACGATACAGAGCWGAGGCGG 3'
   This primer comprises a *Sal*I site (underlined). The 3' part of the primer hybridizes with nucleotides 2027-1998. The C at the 5' end corresponds to nucleotide 2039.

A mixture of both primers D1 and D2 may advantageously be used.

W means A or T. Thus in SEQ ID NO: 17 and 18, W can represent A or T. A mixture can be used. Generally, the primers will be synthesized with degeneracy, and both possibilities will be present.

The reverse primer comprises a restriction site (e.g. a *Sph*I or *Sal*I site) which is also present in the vector so as to allow fusion of the HBV DNA into the vector. Both sites are unique in the vector and in amplicons obtained by PCR with primers C and D).

The use of these particular primers forms a preferred embodiment. However, as mentioned *supra,* the one skilled in the art is able from the present teaching to use other primers or to clone more than two, e.g. 3, genomic fragments with appropriate primer pairs. Use is preferably made in all cases of the forward primers of type A and/or of the reverse primers of type D.

In a second embodiment, the aim is to clone a more-than-full-length HBV genome under the control of homologous enhancer and promoter, thus comprising the HBV nucleotide sequence of enhancer 1 (EN 1 or enh 1, nucleotides 1180 to 1219, H. Norder et al., and GenBank AB048704), and ending on or after a polyA attachment site. Before cloning, preferably two HBV genome fragments have to be amplified.

A first primer pair comprises a forward primer E which preferably is complementary to a consensus or a substantially consensus sequence within the HBV genomes, this primer being designed to be complementary to a region of the HBV genome which is in 5' of enhancer 1, preferably as close as possible of this enhancer.

By way of example, this forward primer E can be as set forth in
SEQ ID NO: 19
   5' TAAACAATGCATGAACCTTTACCCCGTTGC 3'
   This primer comprises a Nsil site (underlined). The primer hybridizes with nucleotides 1125-1154 (H. Norder et al., and GenBank AB048704).

The first primer pair comprises a reverse primer chosen to be complementary to a consensus or a substantially consensus sequence within the HBV genomes. The above described reverse primer D can be used, e.g. either D1 or D2 or D1 and D2, complementary to a region situated in 3' of the polyA attachment site.

Primers E and D allow to amplify a genomic fragment containing the enhancer 1, a unique Ncol site which is situated at the level of the ATG of the X gene, and the polyA attachment site (position 1372-1377 in Norder et al. and GenBank AB048704).

The second primer pair comprises a forward primer and a reverse primer allowing to amplify an about one genome unit containing the same Ncol site, with a forward primer designed to be complementary to a region of HBV DNA which is in 5' with respect to that complementary with the reverse primer of the first primer pair, and a reverse primer designed to be complementary to a region of HBV DNA which is in 3' with respect to that complementary with the forward primer of the first primer pair.

By way of example, use can be made of primers comprising the sequences SEQ ID NO: 20 (forward primer) and SEQ ID NO: 21 (reverse primer) described in the examples, or the following primers:
Forward primer
SEQ ID NO: 22
   5' GCTCTTCTTTTTCACCTCTGCCTAATCATC 3'
   containing a Sapl site; hybridizes with nucleotides 1821 to 1843.
Reverse primer
SEQ ID NO: 23
   5' GCTCTTCAAAAAGTTGCATGGTGCTGGTG 3'
   containing a Sapl site; hybridizes with nucleotides 1825 to 1804.

Preferably, these primers SEQ ID NO: 22 and 23 comprise further nucleotides at the 5' end, in particular 8 or more, e.g. 8 to 15. This is to facilitate the action of the restriction enzyme.

All nucleotide sequences or polynucleotides and primers, as well as primers pairs, described above are also objects of the invention.

In an embodiment, the invention concerns a primers pair for HBV amplification comprising a forward primer comprising (1) a sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12, and (2) a reverse primer comprising a sequence as set forth in SEQ ID NO: 13 or SEQ ID NO: 14 or a mixture of a reverse primer comprising a sequence as set forth in SEQ ID NO: 13 and a reverse primer comprising a sequence as set forth in SEQ ID NO: 14.

In another embodiment, the invention concerns a primers pair for HBV amplification comprising (1) a forward primer comprising a sequence as set forth in SEQ ID NO: 15 or SEQ ID NO: 16 or SEQ ID NO: 19, or a mixture of a forward primer comprising a sequence as set forth in SEQ ID NO: 15 and a forward primer comprising a sequence as set forth in SEQ ID NO: 16, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

The invention also relates to kits for amplifying HBV, comprising two primer pairs according to the invention. In an embodiment, the kit comprises both primers pairs described just before.

In still other embodiment, the invention concerns a primers pair for HBV amplification comprising (1) a forward primer comprising a sequence as set forth in SEQ ID NO: 19, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

In still other embodiments, the invention concerns a primers pair for HBV amplification comprising (1) a forward primer comprising a sequence as set forth in SEQ ID NO: 20 or 22, and (2) a reverse primer comprising a sequence as set forth in SEQ ID NO: 21 or 23.

The invention also relates to a kit comprising both primers pairs described just before.

The one skilled in the art is able to design other primers. Following the same scheme he is also able to amplify more than two fragments, e.g. 3 fragments, using the corresponding number of primer pairs, and to clone the more-than-full-length DNA from these fragments.

And the one skilled in the art is also able to determine the best conditions for the amplifications, depending on the primers which are used. The hybridisation temperature may be determined owing the usual method based on the determination of the dissociation temperature. Also, generally speaking, the amplification may involve a number of cycles, e.g. from 20 to 60, in particular from 30 to 50, preferably about 40 cycles. The Taq polymerase, preferably High Fidelity Taq polymerase (e.g. Expand from Roche Diagnostics) may be used.

In step (c), the product of the amplification is cloned into a vector. This leads to a vector comprising HBV DNA covering the more-than-full-length HBV genome or linear continuous DNA, and elements controlling the synthesis of a pgRNA from the DNA sequence post-cell-transfection. Such an *in vitro* expression vector is also an object of the invention. It is preferably a plasmid.

The vector comprises the elements necessary to govern synthesis of pgRNA from an HBV DNA sequence as defined *supra.* This comprises a promoter able to ensure the transcription of DNA into RNA preferably in eucaryotic cells.

In the first embodiment, the promoter is an heterologous promoter operatively linked to the nucleotide representing the +1 of transcription of the pgRNA, as defined above. The vector called herein type-I vector does not comprise the ATG of the pre-C gene in the 5' part of the cloned HBV DNA sequence.

Potentially, vectors containing an ubiquitous mammalian promoter may be used for constructing a type-I vector. Advantageously, the heterologous promoter is strong which means that transcription level is higher than with the endogenous promoter.

In a preferred embodiment, the heterologous promoter is the early promoter of the cytomegalovirus or CMV-IE (Cytomegalovirus Immediate Early), in particular of human or murine origin, or optionally of any other origin such as rat or guinea pig. The CMV-IE promoter may comprise the proper promoter component, which may or may not be associated with the enhancer component. Use is preferably made of the complete promoter, including the enhancer. Reference may be made to EP-A-260 148, EP-A-323 597, US-A-5 168 062, US-A-5 385 839, US-A-4 968 615 and WO-A-87/03905, Boshart M. et al., Cell., 1985, 41, 521-530.

Other promoters include the human ubiquitine C gene promoter, the promoter of EF-1α gene, the early and late promoters of the SV40 virus (Simian Virus 40), the LTR promoter of the Rous sarcoma virus, cytoskeleton gene promoters, such as, the desmin promoter (Kwissa M. et al., Vaccine, 2000, 18(22), 2337-2344) or the actin promoter (Miyazaki J. et al., Gene, 1989, 79(2), 269-277), e.g. of the chicken β-actin gene, which is also a preferred choice (Fregien N, Davidson N. Gene 1986 48, 1-11).

Use is preferably made of an enhancer combined to a promoter. In a preferred embodiment, the CMV-IE enhancer is combined to a promoter of another origin, e.g. with the actin promoter, in particular the chicken β-actin gene.

By equivalence, the sub-fragments of these promoters, maintaining adequate promoter activity, are included in the present invention: e.g. the truncated CMV-IE promoters according to WO-A-98/00166. The notion of promoter according to the invention therefore includes the derivatives and sub-fragments maintaining adequate promoter activity, preferably substantially similar to that of the proper promoter from which they are derived. With regard to CMV-IE, this notion includes the proper promoter part and/or the enhancer part and the derivatives and sub-fragments.

As explained above, it is preferred to fuse the natural +1 of transcription of the HBV pgRNA to the +1 of transcription determined by the promoter contained in the vector or plasmid used for the cloning. To achieve this fusion at molecular level, the promoter may be modified by the insertion (mutagenesis), between the TATA box (or equivalent) of the basic promoter and its +1 of transcription, of a restriction site which preferably is not present in any of the known complete HBV sequences (e.g. one of the previously mentioned *Not*I, *Asc*I, *Pac*I, *Pme*I and *SSe*8387 I restriction sites). The restriction site has to be the same than the one present in the primer A. See the examples.

The cloning necessitates the ligation of the HBV DNA fragments together, and insertion into the vector. This technique is well known to one skilled in the art using suitable sets of restriction enzymes and restriction sites. Preferably step (c) is a one-step cloning. And the primers specifically disclosed herein allow a rapid one-step cloning.

Using a type A primer, the vector comprises:
(a) a linear continuous or more-than-full-length HBV DNA sequence as defined *supra* under the first embodiment (for sequence under the control of a heterologous promoter), able to produce a pgRNA according to the invention
(b) a promoter modified in 5' by the presence of a restriction site in 5' of the +1 of transcription, with the +1 of transcription of the DNA sequence and of the promoter being fused, the promoter controlling the synthesis of a pgRNA from the DNA sequence post-cell-transfection. This restriction site is preferably not present in any of the known complete HBV sequences and can be e.g. *Not*I*, Asc*I, *Pac*I, *Pme*I or *SSe*8387 I. In some particular embodiments, the promoter sequence comprises a sequence chosen among SEQ ID NO: 24 to 29 (end part of the specification), after putting the restriction site as indicated.

The vector may also comprise baculovirus sequences, allowing one to transfer the vector in baculovirus. These sequences are sequences flanking a baculovirus genome region, e.g. gene, which is not essential for viral replication in cell culture. These sequences allow recombination between the vector and the baculovirus genome, so that the cassette containing the promoter and the HBV unit becomes inserted in the baculovirus genome upon transfer of the vector into the baculovirus.

As it is well know, the vector can also comprise a replication origin and a selection marker, such as a resistance gene, e.g. a blasticidin resistance gene. This could be helpful in the course of generating stable cell lines, to select the cells containing the vector.

A vector comprising the baculovirus sequence and the selection marker is contemplated herein. The same vector could thus be used either to transfect cells, or to transduce cells via baculovirus, or to produce a stable line cell. In particular, once an interesting clone has been found from a patient sample, it is possible to produce a stable cell line in view of screening of efficient drugs.

The synthesis of a pgRNA initiates in the cells the HBV intracellular cycle with viral gene expression, including synthesis of intracellular replicative intermediates (which comprise single stranded DNA, double stranded linear DNA, relaxed circular DNA and the minor species of replicative intermediates, RNAs), gene expression and virus production.

Such vectors are also objects of the invention. Also, the promoters modified as described herein, including in the examples, constitute other objects of the invention.

In the second embodiment, the promoter is an homologous promoter, which is present in the cloned HBV DNA. A type-II vector is produced in a two step procedure. First, the amplicon obtained with the first set of primers, e.g. E-D, is digested and inserted into the vector. Then, the amplicon obtained with the second set of primers, e.g. P1-P2, is digested by Sapl, ligated (circularized) using a ligase, then digested with Ncol and inserted in the Ncol site present in the cloned E-D fragment.

This strategy produces a vector comprising :
- a linear continuous or more-than-full-length HBV DNA sequence according to the second embodiment
- wherein the homologous enhancer and promoter controls the synthesis of a pgRNA from the DNA sequence post-cell-transfection.
   More precisely, the vector initiates synthesis of pre-C RNA and of pgRNA. Such vectors are also objects of the invention.

In step (d), cultured susceptible cells can be transfected or transduced with the vector according to the invention.

In one embodiment the susceptible cells, in particular eucaryotic cells such as hepatocytes, including cell lines, in particular cell lines from hepatocyte origin, e.g. hepatoma cell lines, e.g. Huh7 (Nakabayaski, H. et al.. Cancer Res.1982 42,3858-3863), HepG2 (available from the ATCC, access number HB-8065) are transfected with the vector.

At step (e), the cells are cultured to allow the vector to synthesize pgRNA and possibly initiate the HBV intracellular cycle as described previously.

In step (g), replication capacity of HBV is evaluated. This may involve measuring the level of nucleic acid synthesis, protein synthesis and/or virus production.

In one embodiment, the transfected cells may be used for studying the replication capacity of the HBV on the cells. This may involve quantification of viral DNA by molecular biology techniques, e.g. by Southern blot or by real time amplification specifically of the synthesized nucleic acids.

In another embodiment comprising step (f), these transfected cells are used for studying the effect of antiviral agents. In step (f), when a pharmaceutical product, in particular the antiviral agent, is used, it is added to the cultured transfected cells. In an embodiment, a part of the cultured transfected cells is kept as a control, say without being subjected to the pharmaceutical product, in particular the antiviral agent, in order to have a comparison basis. In another embodiment, one or more parts of the cultured transfected cells is/are subjected to another pharmaceutical product(s), in particular antiviral agent(s), which allows comparison between different pharmaceutical product(s), in particular antiviral agent(s). And in this embodiment, a control without any treatment may be added.

In particular embodiments, the antiviral agents used are chosen among lamivudine, adefovir dipivoxil, entecavir, emtricitabine, clevudine, telbivudine, tenofovir, beta-L-FD4c and any combinations thereof.

In still another embodiment, the vector is transferred inside a baculovirus upon infection of an appropriate system of cells such as insect cells, and propagated. One may use for example the Baculovirus Autographa californica Nuclear Polyhedrosis Virus AcNPV. One may use for example Spodoptera frugiperda Sf9 (ATCC CRL 1711 deposit) as insect cells. The baculovirus containing the vector is then used for transducing eucaryotic cells such as hepatoma cells, e.g. Huh7 cells, where the vector can initiate pgRNA synthesis. Then in step (f), a pharmaceutical product, in particular an antiviral agent, is added to the cultured transduced cells. In an embodiment, a portion of the cultured transduced cells is kept as a control, say without being subjected to the pharmaceutical product, in particular the antiviral agent, in order to have a comparison basis. In another embodiment, one or more portions of the cultured transduced cells is/are subjected to another pharmaceutical product(s), in particular antiviral agent(s), which allows comparison between different pharmaceutical product(s), in particular antiviral agent(s). And in this embodiment, a control without any treatment may be added.

In still another embodiment, the vector is used for generating cell lines which become able to constitutively produce HBV virions. As mentioned above, the vector may comprise a selection marker. Cell lines such as cell lines from hepatocyte origin, e.g. hepatoma cell lines, e.g. Huh7, HepG2 are transfected with the vector as mentioned above and the cells selected owing the marker are recovered as stable cell line comprising the vector according to the invention. These stable cell lines are also an object of the invention.

As explained before, the present invention results in producing HBV pgRNA, intracellular replicative intermediates, and HBV viral particles. It is thus possible in step (g) to follow different parameters, in the presence or in the absence of pharmaceutical product, in particular an antiviral agent or candidate.

In a first embodiment, the level of HBV DNA synthesis in the cells is evaluated and possibly compared. A general method provides the disruption of the cells, e.g. by physical and/or chemical treatment, for instance lysis, e.g. with Tris-EDTA and Nonidet P-40. The recovered sample is centrifuged, the recovered supernatant is subjected to DNase to eliminate transfected DNA, whereas the synthesized DNA or being synthesized is encapsidated. Dnase activity is neutralized using EDTA or similar, then DNA is liberated from the capsides using proteinase K or similar. DNA is extracted using e.g. phenol-chloroform, and precipitated. Then measuring of the level of DNA may be made using any known method, in particular Southern blot or PCR. IC₅₀ and IC₉₀ may be calculated.

In a second embodiment, the level of virion DNA in the extracellular medium or supernatant is quantified and possibly compared using molecular biology techniques, such as dot blot, Southern blot and quantitative PCR. IC₅₀ and IC₉₀ may be calculated (inhibition Concentration 50 % or 90 %, say dose of drug which induce a 50 % or 90 % decrease of the measured parameter).

Comparison of the results with standards can be done.

An object of the invention is thus such phenotypic assay method to test the susceptibility of HBV isolates with respect to drugs. In particular, this object is applied to the determination of the susceptibility of the viral strains (e.g. the quasi-species) present in a patient to the available drugs, and then it is possible to adapt antiviral therapy to the virological situation. In a particular embodiment, the method of the invention comprises the following steps:
1) DNA extraction from serum of a patient
2) PCR amplification
3) Cloning into type-I vectors
4) Transfection of the vectors in cells, e.g. Huh7 cells, and transfer in 96 well plaques
5) Treatment with antiviral to be evaluated
6) Quantitative PCRs and determination of IC₅₀ and IC₉₀
7) Comparison with standards.

Another object of the invention is to use this phenotypic assay method for testing the antiviral activity of current and future drugs to HBV strains or to HBV field isolates. In a particular embodiment, the method of the invention comprises the following steps:
1) Possibly DNA extraction from serum of a patient or from a viral culture
2) PCR amplification
3) Cloning into type-I or type-II vector
4) Transfection of the vectors in cells, e.g. Huh7 cells, or transduction of cells via baculovirus, or production of a stable cell line, and e.g. transfer in 96 well plaques
5) Treatment with antiviral to be evaluated
6) Quantitative PCRs and determination of IC₅₀ and IC₉₀
7) Comparison with standards.

In the method according to the invention, when all or part of the clones obtained from one sample (e.g. the quasi-species) is used to transfect or transduce cells, a multiclonal analysis is made.

The invention thus allows the screening of drugs. A further object of the invention consists in the pharmaceutical compositions useful against HBV that have been prepared after selection of an active agent with the method of the invention. Another object of the invention is a method for producing a pharmaceutical composition to be used as antiviral drug against HBV, comprising the assay method according to the invention, followed by the admixture of the selected active agent with a pharmaceutically acceptable vehicle or excipient.

Another object of the invention is the method to rapidly clone HBV DNA from patient samples, as disclosed herein.

To perform the teaching disclosed herein, the one skilled in the art may refer to the usual methods of molecular biology, recombinant DNA, microbiology, as illustrated for example in : Sambrook and Russel, Molecular Cloning: A Laboratory Manual, Third Edition (2001) Cold Spring Harbor Laboratory Press, Cold Spring Harbor, New York (herein "Sambrook et al., 1989") ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; B. Perbal, A Practical Guide To Molecular Cloning (1984) ; F.M. Ausubel et al. (eds.), Current Protocols in Molecular Biology, John Wiley & Sons, Inc. (1994).

The present invention is additionally described by the following illustrative, non-limiting examples, which refer to the attached drawings.

### Brief description of the drawings:

Figure 1 is a map of the open circular HBV genome with its open reading frames represented. The different primers used in this study are indicated

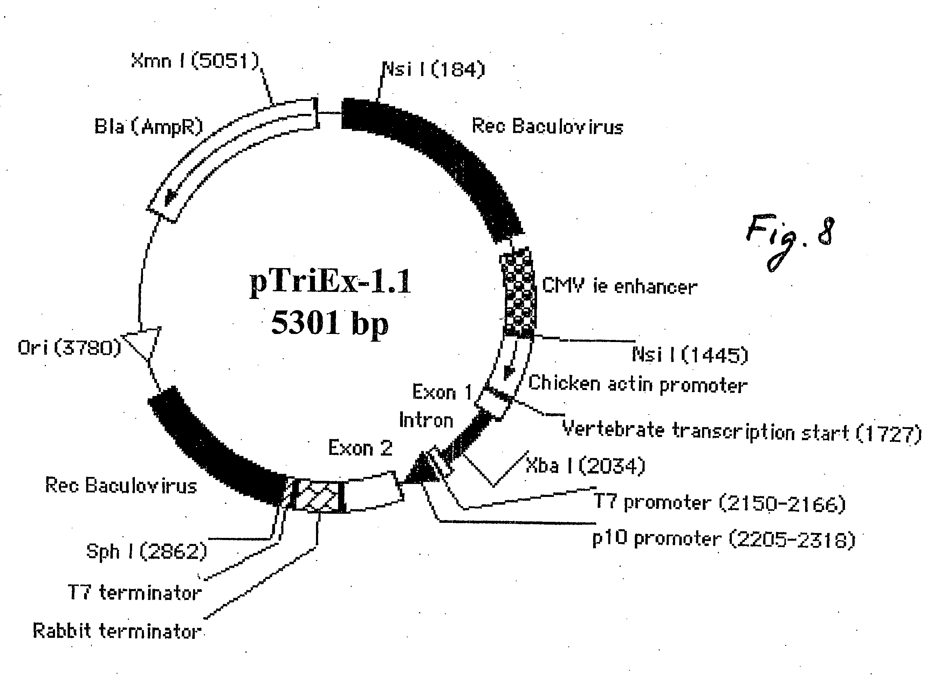

Figure 2 represents a schematic map of pTriEX-1.1 and derived plasmids pTriEX-Mod and pTriEX-Bsd are given as well as the detailed sequence of the basic core promoter (BCP) of the chicken β-actin gene. The modification of the BCP by insertion of a Not I site is shown. The whole map of circular pTriEX-1.1 is represented on Figure 8.
Figure 3 presents the sequence of the forward primer A used for PCR amplification and the primer binding site at the triple-stranded region of the HBV genome.

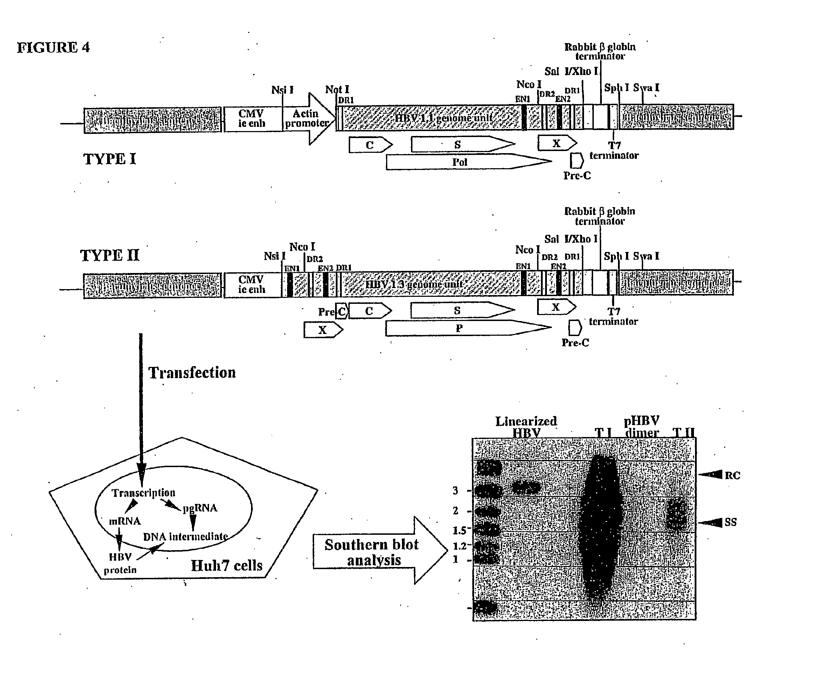

Figure 4 presents type-I *and type-II* vectors, transfection of Huh7 cells with either type-I, type-II, or pHBVdimer plasmids, and the result of a southern blot analysis of intracellular replicative HBV DNA intermediates. Lanes TI, TII, and pHBVdimer correspond to DNA extracted from viral core particles derived from Huh7 cells that were transfected respectively with type-I, type II, and pHBVdimer circular plasmids. Lane denoted linearized HBV correspond to a 3.2 kb linear double-stranded form of HBV DNA. The position of the relaxed circular (RC) and the single stranded (SS) forms of the HBV DNA genome are indicated on the right of the autoradiography. DNA size markers are indicated on the left.

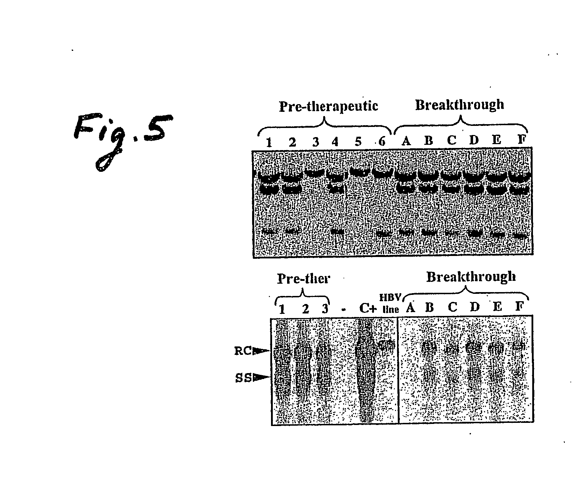

Figure 5 presents cloning performance and replicative status of clones obtained. (A) HBV DNA extracted from serum of patient before therapy (pre-therapeutic) or when viral resistance appear (breakthrough), was PCR amplified using two pairs of primers, i.e. A-B and CD, and cloned in type-I vectors. Positive clones present the restriction pattern found in lanes 1,2, 4, A-F of the ethidium-stained agarose gel presented. Positive clones were used for the transfection of Huh7 cells. A control type-I plasmid carrying a wild type genotypes D HBV genome was used as control (lane C+). Southern blot analysis of intracellular replicative HBV DNA intermediates was performed 4 days post transfection. The position of the relaxed circular (RC) and the single stranded (SS) forms of the HBV DNA genome are indicated on the right of the autoradiography.

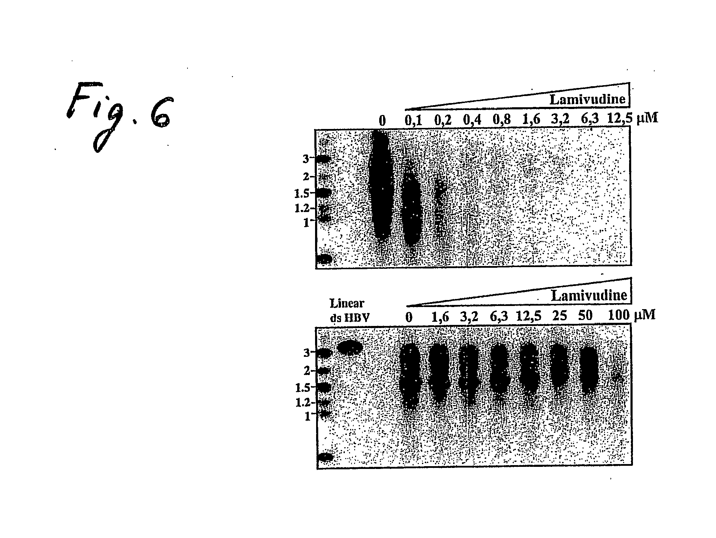

Figure 6 presents two phenotypic drug susceptibility profile after southern blot analysis. Two mixed population of type-I vectors obtained from the cloning of lamivudine-sensitive (top panel) and lamivudine-resistant (botttom panel) HBV genomes have been used for the transfection of Huh7 cells. Cells were treated during 5 days, starting at day 3 post-tranfection, with increasing concentration of lamivudine (shown on the top of each autoradiography). A Southern blot analysis of intracellular replicative HBV DNA intermediates was performed. The autoradiographies corresponding to a sensitive profile (top panel) and a resistant profile (bottom panel) are shown.

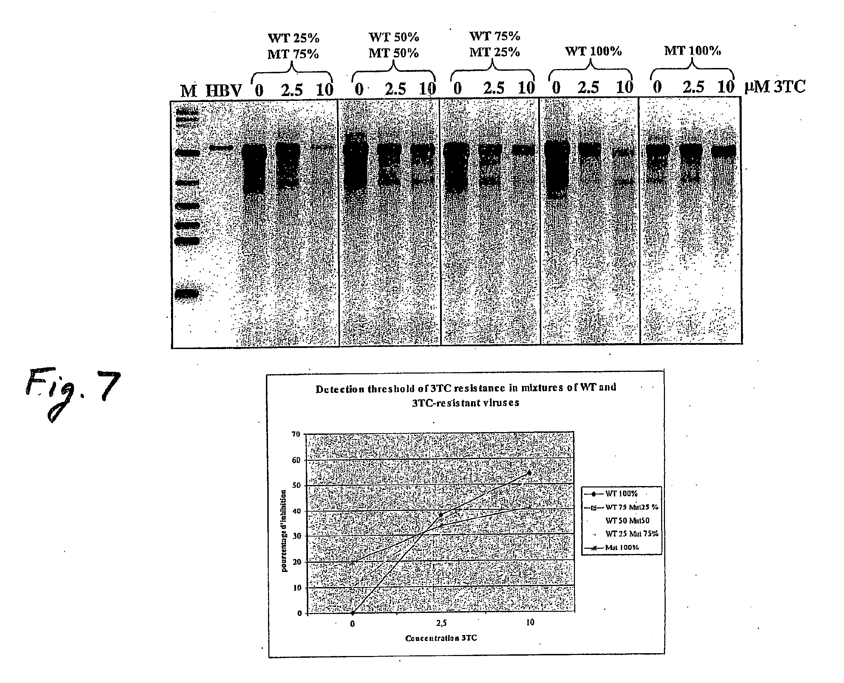

Figure 7 presents sensitivity profiles of the phenotypic assay. Mixtures of type-I clones containing either wild type derived HBV genomes or 3TC-resistant derived HBV genomes (containing mutation L528M and M552V) were used for the transfection of Huh7 cells. Cells were left untreated or treated during 5 days, starting at day 3 post-tranfection, with two concentration of lamivudine, i.e. 2.5 or 10 µM. A Southern blot analysis of intracellular replicative HBV DNA intermediates was performed. The composition of the mixture used is indicated on the top of the autoradiography. A phosphoimager analysis of the blot was performed and the results are presented on the graph underneath where the percentage of the inhibition of the replication is represented in function of the amount of lamivudine used.
Figure 8 presents TriEX-1.1 plasmid.

### Examples:

### Materials and Methods

**Plasma samples**. Plasma samples, obtained from chronically HBV-infected individuals, were stored at -20°C until analysis. A reference serum, ACCURUN® 325 HBV DNA positive control manufactured by BBI Diagnotics (Boston, MA), was used for the setting up of some experiments.

**Antiviral drugs**. β-L-(-)-2',3'-dideoxy-3'-thiacytidine (3TC, lamivudine), from Glaxo SmithKline.

### Sample preparation and PCR amplifications.

Viral DNA was extracted from 200 µl of serum using three different commercialized kits (Master pure™ Complete DNA and RNA purification kit [Epicentre, Madison, WI], High pure PCR template preparation kit [Roche Diagnostics, Mannheim, Germany], and QIAamp™ UltraSens virus kit [Qiagen, Hilden, Germany]) according to manufacturer's instructions. The final volume of elution or resuspension was 60 µl and 5 to 10 µl were used for subsequent polymerase-chain-reaction (PCR) amplifications. All PCR were performed using a "hot start" procedure with the Expand high fidelity PCR system (Roche Diagnostics, Mannheim, Germany) according to manufacturer's instruction. Four sets of primers were used.

Full length HBV was amplified according to the method described by Günther *et* a*l* (J. Virol. 1995, 69, 9: 5437-5444 and J Clin. Microbiol. 1998, 36, 2: 531-538), with the forward primer P1 SEQ ID NO: 20 (5'-CCGGAAAGCTTAT GCTCTTCTTTTTCACCTCTGCCTAATCATC-3') containing *Hind*III/*Sap*I sites, and the reverse primer P2 SEQ ID NO: 21 (5'-CCGGAGAGCTCAT GCTCTTCAAAAAGTTGCATGGTGCTGGTG-3') containing *Sac*l/*Sap*l sites.

A long PCR product of approximately 2800 bp was amplified by 40 cycles (hot-start; 94°C for 40 s, 55°C for 1 min, 68°C for 3 min with an increment of 2 min after each 10 cycle) with a forward primer A (5'-TGCGCACCGCGGCCGC GCAACTTTTTCACCTCTGCC-3'; SEQ ID NO:1) containing a *Not*I site (see Figure 3) and a reverse primer B (B1 for amplification of genotypes A, B, D, E, and G: 5'-GGCAGCACASCCTAGCAGCCATGG-3', SEQ ID NO:13, or B2 for amplification of genotypes C and F: 5'-GGCAGCACASCCGAGCAGCCATGG-3'; SEQ ID NO: 14) containing a *Nco*l site.

A short PCR product of approximately 665 bp was amplified by 40 cycles (hot-start; 94°C for 30 s, 50°C for 30 s, 72°C for 45 s with an increment of 5 s after each cycle starting from cycle 11) with a forward primer C (C1 for amplification of genotypes A, B, D, E, and G: 5'-ACMTCSTTTCCATGGCTGCTAGG-3', SEQ ID NO:15, or C2 for amplification of genotypes C and F: 5'-ACMTCSTTT CCATGGCTGCTCGG-3'; SEQ ID NO: 16) containing a *Nco*l site and a reverse primer D (D1 containing a *Sph*I site: 5'-CTAAGGGCATGCGATA CAGAGCWGAGGCGG-3', SEQ ID NO:17, or D2 containing a *Sal*I site: 5'-CTAAGGGTCGACGATACAGAGCWGAGGCGG-3'; SEQ ID NO: 18).

An other PCR product of approximately 900 bp was amplified by 40 cycles (hot-start; 94°C for 30 s, 50°C for 30 s, 72°C for 45 s with an increment of 5 s after each cycle starting from cycle 11) with a forward primer E (5'-TAAACAATGCATGAACCTTTACCCCGTTGC-3'; SEQ ID NO: 19) containing a *Nsi*l site and the reverse primer D. The location of these primers in respect with the HBV genome is indicated on Figure 1.

### Cloning of more-than-full-length HBV genome into two different types of vectors.

The pTriEX-1.1 vector (Novagen Inc, Madison, WI., Figure 2), containing multiple promoters and flanking baculovirus sequences, was adapted to the cloning strategy by two modifications to derive pTriEX-Mod. First the *Nsi*I site at position 184 was removed, rendering unique the other *Nsi*I site at position 1445, which is located between the CMV enhancer and the actin basic promoter. Second, a unique *Not*I site was added at position 1717, which is just upstream to the vertebrate transcription start (position 1727) of the vector (Figure 2). The plasmid pTriEX-Mod was further modified by cloning at *Mlu*I site (position 399 in pTriEX-Mod), of the blasticidin cassette that was PCR amplified from pUB-Bsd (Invitrogen Ltd, Paisley, U.K.) comprising the blasticidin gene under the control of human ubiquitin C promoter.

The final vector, pTriEX-Bsd, was used when both transfer into baculoviruses and generation of persistently-HBV-expressing cell line were needed. PCR products generated with primers A-B and E-D (or C-D) were digested by *Not*l/*Nco*l and *Nco*I/*Sal*I respectively and cloned, in one step using standard procedures [Sambrook, J. and D.W. Russell, Molecular cloning : a laboratory manual. 3rd ed. 2001, Cold Spring Harbor, N.Y.: Cold Spring Harbor Laboratory Press. 3 v.], between *Not*l/*Xho*l sites of pTriEX-Mod to derive type-I plasmids (Figure 4).

Type-I vector comprising about 1.1 genome unit enables, post-transfection, the synthesis of pre-genomic HBV RNA (pgRNA) under the control of a chicken actin promoter.

Alternatively, PCR product generated with primers E-D was digested by *Nsi*I/*Sal*I and inserted between *Nsi*I/*Xho*I sites of the vector. This intermediate plasmid was subsequently used for the cloning of one HBV genome unit into the unique *Nco*I site located at the level of the ATG of the gene X of HBV. The insert (one HBV genome unit) was obtained by the successive treatments of a P1-P2 full-length PCR product with *Sap*l followed by T4 ligase (circularization) then *Nco*l digestion. This led to type-II vector comprising about 1.3 genome unit, which enables post transfection the synthesis of pgRNA under the control of natural HBV promoter (Figure 4).

### Transfection of HBV DNA.

All cloned DNA were purified by the Qiagen technology, based on silica columns (Qiagen Inc., Valencia, California, USA). Transfection of plasmids into Huh7 cells was performed with Fugene-6 reagent (Roche Diagnostics, Mannheim, Germany) according to manufacturer's instruction. Briefly, Huh7 cells were seeded at a density of 7 x 10⁴ cells/cm² and transfected 12 hours later with 150 ng of plasmid per cm². The medium was changed one hour before transfection, then every day in case of treatment with antiviral.

### Purification of HBV DNA from intracellular core particles and Southern blot analysis.

The cells were washed once with PBS then lysed in 1 ml of lysis buffer (50mM Tris-HCl [pH 8], 1mM EDTA, 1% Nonidet P-40) per 35 mm diameter dish. The lysate was incubated on ice for 15 min, and nuclei were pelleted by centrifugation for 1 min at 14,000 rpm. The supernatant was adjusted to 10mM MgCl₂ and treated with 100 µg of DNAse for 1 hour at 37°C. The DNAse activity was stopped by addition of EDTA to a final concentration of 25mM, then proteins were digested with 0.5 mg/ml of proteinase K for at least 2 hours in presence of 1% SDS and 100 mM NaCl. Nucleic acids were phenol-chloroform extracted and iso-propanol precipitated after addition of 10 µg of tRNA. DNAs were separated on 1.2% agarose gel, blotted onto positively-charged Nylon membranes, and hybridized with a ³²P-labelled full-length HBV probe as described previously [Seigneres, B., et al., Hepatology, 2002. 36(3): p. 710-22.].

### Purification of HBV DNA from extracellular viral particles and quantitative PCR analysis.

Viral DNA was extracted from cell culture medium using either the commercialized QlAamp™ UltraSens virus kit (Qiagen, Hilden, Germany) or by the following procedure. One ml of clarified culture medium was supplemented up to 10%final with polyethylene glycol (PEG), then centrifugated 1 hour at 14,000 rpm in a bench centrifuge at 4°C. The pellet was resuspended in 1 ml of lysis buffer and proceeded as described above for the isolation of HBV DNA from cytoplasmic core particles.

### Results

### Extraction of viral DNA from sera and PCR performance.

Several kits are available for HBV DNA isolation from serum. Three of them have been tested to optimize the quantity/quality of the isolated HBV DNA that will be subsequently used as PCR-template. Some of them imply column-based purification while the other is based on differential precipitation of proteins and nucleic acids. Two hundred microliter of a reference serum were extracted using these different approaches and real time PCR was performed to quantify the amount of DNA obtained. The "Master pure™ Complete DNA and RNA purification kit" yielded higher DNA recovery, with a vDNA (viral DNA) concentration of 2.73 10⁴ copies/µl, compared to the two other column-based techniques, which yielded a concentration of 1.08 10⁴ copies/µl (High pure PCR template preparation kit) and 9.77 10³ copies/µl (QIAamp™ UltraSens virus kit) respectively. To determine whether the quality of the DNA obtained by these techniques was comparable, dilutions (1/10^{th}) of these DNA were used for setting up PCR reactions. Primers P1 and P2, derived from the Gunther's method, were first used for finding out the threshold of the PCR. The PCR was performed using the Expand high fidelity PCR system and the PCR conditions described previously [Gunther et al. 1998 and 1995 cited above].

The vDNA obtained with QlAaMp™ UltraSens virus kit was the best in terms of quality of the DNA, as a PCR product was visible in an ethidium bromide-stained gel when as low as 5 copies of genome were used as initial template. According to the extraction procedure used (see Materials and Methods), these 5 copies corresponded to a theoretical serum titer of 300 genomes/ml. To obtain the same amount of amplicon using DNA extracted with the Master pure™ Complete DNA and RNA purification kit, 20 copies of genome/reaction (corresponding to a theoretical serum titer of 1200 genomes/ml) were necessary, indicating a lower quality of this DNA.

### Construction of type I vectors:

As described above, a pTriEx-1.1 (Novagen) plasmid was engineered by introduction of a *Not*I site at the level of the +1 of transcription of the chicken actin promoter (Figure 2). No modification of the promoter activity was observed for the modified plasmid, i.e. pTriEX-Mod, as measured by reporter gene expression. Primer A located at the +1 of transcription of the pgRNA contains a *Not*I tail (Figure 3). This primer A was used in combination with the reverse primer B located at the level of the ATG of HBV X gene to set up a PCR reaction. A conserved *Nco*I site, essential for the cloning strategy, overlaps this ATG.

The PCR products obtained with the two pairs of primers A-B and C-D2 (or E-D2) were digested with *Not*I/*Nco*I and *Nco*I/*Sal*I and simultaneously cloned into pTriEX-Mod to derive types-I vectors (Figure 4). This fusion resulted in the exclusive production in transfected-hepatoma cells of HBV pgRNA at a steady level regardless of the information contained in the 1.1 unit of genome cloned altogether. In average, between 50 and 75% of the clones obtained presented the expected restriction pattern and more than 75% of these clones led to formation of DNA intermediate upon cell transfection as verified by Southern blot analysis (Figure 5). Taken together, these data indicate that when 40 cycles of PCR are used for generating inserts A-B and C-D, between 40% to 65% of the resulting types I clones have both the expected restriction pattern and are replication-competent.

Within hepatoma cells, type-I vector enables the synthesis of pgRNA at a level that is determined by the chicken actin promoter strength. This type of vector is of interest for the analysis of event of the viral cycle occurring post-transcription. It can be use for instance for characterizing mutations in the polymerase gene, which are associated with resistance to nucleoside/nucleotide analogues.

### Construction of type II vectors:

A second cloning strategy was designed to construct type-II vectors in which the synthesis of pgRNA is kept under homologous promoter. Two cloning steps were necessary to derive these vectors containing 1.3 unit of HBV genome.

First the PCR product of 900 bp obtained with primers E-D2 was double digested with *Nsi*I/*Sal*I and cloned into pTriEX-Mod opened by *Nsi*l and *Xho*l. The unique *Nco*l site of the derived intermediate plasmid was then used for cloning one HBV genome unit which was generated by the successive treatment of a P1-P2-derived PCR product with *Sap*I, then T4 DNA ligase, and *Nco*I (Figure 4).

The type-II vector allows to study transcriptional properties of HBV mutants, such as core-promoter mutants, or to evaluate in which the intrinsic level of synthesis of pgRNA had an impact on the phenotype observed.

### Comparison of the amounts of the intracellular replicative intermediates observed in hepatoma Huh7 cells after transfection with type I, type II, head-to-tail tandemerized HBV genomes or HBV genomes with Sapl sticky ends.

The genome of a well defined HBV genotypes D (serotype ayw) was cloned i) into type-I and type-II vectors, ii) as head-to-tail tamdem into pUC vector, or iii) according to Gunther's method into pUC vector. These four types of construction were used for transfecting hepatoma Huh7 cells.

The HBV genome cloned by Gunther's method was first released by *Sap*l digestion before the transfection. The same amount of DNA (equivalent HBV genome) was used. As expected, due to presence of the strong actin promoter, the type-I vector gave rise to the highest level of intracellular replicative intermediates (Figure 4), thus confirming the usefulness of such vector for post transcriptionnal phenotypic studies. Type-II vector gave rise to higher level of intracellular replicative intermediates than both head-to-tail tandem and Günther's type constructions, suggesting that type-II vector may be used as alternative of type-I vector in a phenotypic assay.

### Description and calibration of drug susceptibility assay.

Huh7 cells were seeded in 35mm Ø dishes (7. 10⁵ cells/dish) and allowed to adhere for 6 hours. Cells were transfected with 1.5 µg of a mixture of at least 10 clones of type-I vectors obtained from the cloning of HBV from a serum to be tested.

This multiclonal approach was meant to mirror the viral quasispecies. The day after, the medium was replaced by fresh medium containing different concentrations of the drug to be tested (e.g. lamivudine in the example given). Treatment with drug was renewed everyday for four days. Then cells were lysed as described above and a Southern blot analysis was performed to measure the amount of intracellular replicative intermediates synthesized under drug pressure and determine the IC₅₀. Experiments were performed in triplicate. On the example given (Figure 6), for a wild type HBV genomes population the IC₅₀ of lamivudine was estimated less than 100 nM while it increased to more than 50 µM for a viral population cloned from patient who had a viral breakthrough under lamivudine (3TC) treatment.

To get insight on the sensitivity of the assay, incremental mixtures of plasmids containing either wild type derived HBV genomes or 3TC-resistant derived HBV genomes (containing mutation L528M and M552V [Seigneres et al., 2002 supra]) were used for cell transfection. The results of a Southern blot as well as the scan-densitometry analysis are presented on Figure 7. These data demonstrate that the assay readily distinguished mixtures that comprised 25, 50, or 75% resistant virus from the samples with 100% drug sensitive or 100% drug-resistant HBV genomes.

Such modelisation could allows one to compare the IC₅₀ of a treated patient sample to detect a possible breakthrough.

Similar assay has been performed with the following drugs: emtricitabine, clevudine, adefovir dipivoxil, tenofovir disoproxil and beta-L-FD4C, demonstrating that the method of the invention may be used in phenotypic assay to assess drug susceptibility of HBV clones.

### Various promoters useful in the invention

Are represented below a partial nucleotide sequence of known promoters. The 3' terminal part thereof is represented with the +1 of transcription in bold and underlined (the nucleotide on the left is noted -1) and the TATA box also in bold when present. For each promoter, a location for creating a restriction site is indicated, as well as the A primer(s) which can be used therewith, in accordance with the first embodiment of the invention.

Basic promoter sequence of Chicken β actin gene:
SEQ ID NO: 24
CGGCCC**TATAAAA**AGCGAAGCGCGCGGCGGGCG**G**GAGTCGCT

| Restriction site | Will replace nucleotides | Primer A SEQ ID NO: |
|---|---|---|
| *Not*I | -10 to -3 | 1 |
| *Asc*I | -8 to -1 | 2 |
| *Sse*8387 I | -10 to -3 | 3 |
| *Pme*I | -8 to -1 | 4 |

Basic promoter sequence of CMV-IE gene:
SEQ ID NO: 25
**TATATAA**GCAGAGCTCGTTTAGTGAACCGTC**A**GA

| Restriction site | Will replace nucleotides | Primer A SEQ ID NO: |
|---|---|---|
| *Not*I | -9 to -2 | 5 |
| *Asc*I | -8 to -1 | 2 |
| *Sse*8387 | -9 to -2 | 6 |
| *Pme*I | -8 to -1 | 7 |

Basic promoter sequence of human ubiquitin C (hUb) gene:
SEQ ID NO: 26
**TATATAA**GGACGCGCCGGGTGTGGCACAGCT**A**GT

| Restriction site | Will replace nucleotides | Primer A SEQ ID NO: |
|---|---|---|
| *Not*I | -11 to -4 | 1 |
| *Asc*I | -9 to -2 | 8 |
| *Sse*8387 l | -9 to -2 | 6 |

Basic promoter sequence of human EF-1α gene:
SEQ ID NO: 27
**TATATAA**GTGCAGTAGTCGCCGTGAACGTT**C**TT

| Restriction site | Will replace nucleotides | Primer A SEQ ID NO: |
|---|---|---|
| *Not*I | -9 to -2 | 1 |
| *Asc*I | -10 to -3 | 9 |
| *Sse*8387 I | -10 to -3 | 10 |

Basic promoter sequence of early promoter of SV40 (GC rich -TATA less promoter):
SEQ ID NO: 28
GACTAATTTTTTTTATTTATGCAGAGGCC**G**AGGC

| Restriction site | Will replace nucleotides | Primer A SEQ ID NO: |
|---|---|---|
| *Not*I | -9 to -2 | 1 |
| *Asc*I | -9 to -2 | 11 |
| *Sse*8387 I | -10 to -3 | 3 |

Basic promoter sequence of RSV (Rous Sarcoma Virus) LTR:
SEQ ID NO: 29
TATTTAAGTGCCTAGCTCGATACAATAAAC**G**CC

| Restriction site | Will replace nucleotides | Primer A SEQ ID NO: |
|---|---|---|
| *Pme*I | -8 to -1 | 7 |
| *Pac*I | -9 to -2 | 12 |
| *Sse*8387 I | -9 to -2 | 6 |

Each sequence of basic promoter modified by any one of the restiction sites as indicated in the attached table is an object of the invention, as well as each promoter containing that sequence so modified, and each vector containing that promoter.

## Claims

1. A method for measuring the replication capacity of HBV, e.g. HBV present in a biological sample, possibly in the presence of a pharmaceutical product, preferably an antiviral agent, the method comprising:
(a) possibly extracting nucleic acids contained in the biological sample;
(b) PCR amplifying HBV nucleic acids using at least two primer pairs selected so as to obtain at least two different amplified HBV genomic fragments which upon assembly represent a linear continuous DNA sequence transcriptable in pgRNA ;
(c) cloning the fragments obtained under (b) into a vector under the control of an heterologous promoter, so producing a vector containing a linear continuous DNA sequence transcriptable in pgRNA under control of said promoter,
(d) transfecting or transducing susceptible cells with the vector;
(e) culturing the transfected or transduced cells in conditions allowing synthesis of HBV pgRNA from the cloned HBV DNA;
(f) possibly treating the cultured cells with the pharmaceutical product, in particular antiviral agent; and
(g) determining the replication capacity of the HBV, possibly incidence of the pharmaceutical product, preferably antiviral agent, on viral gene expression and/or viral replication,
wherein in step (b) use is made of a primer pair according to claim 32 and a primer pair according to claim 33.

2. The method according to claim 1, wherein the continuous DNA sequence comprises HBV nucleotides (from 5' to 3') :
- an about 1 genome unit starting in 5' from and including the nucleotide representing the +1 of transcription to the first nucleotide in 5' of the ATG of the pre-C gene, plus
- a sub-genomic fragment starting from and including the A of the ATG of the pre-C gene and extending to and including the polyA attachment site,
wherein the linear continuous DNA sequence does not comprise at its 5' end the
ATG of the pre-C gene.

3. The method according to claim 2, wherein the continuous DNA sequence comprises from 5' to 3' nucleotides 1818 to 1813 and 1814 to 1960 of an HBV genomic sequence aligned with the sequence as set forth in GenBank AB048704.

4. The method according to claim 2, wherein the continuous DNA sequence comprises from 5' to 3' nucleotides 1816 to 1813 and 1814 to 2016 of an HBV genomic sequence aligned with the sequence as set forth in GenBank AB048704.

5. The method according to any one of claims 2 to 4, wherein in step (b), use is made of a first primer pair and of a second primer pair, comprising each a forward primer and a reverse primer designed to amplify respectively:
- a first fragment comprising a HBV DNA sequence comprising at its 5' end the nucleotide representing the +1 of transcription
- a second fragment comprising a HBV DNA sequence comprising at its 3' end the polyA attachment site
the 3' end of the first fragment and the 5' end of the second fragment being overlapping and preferably comprising a restriction site in the overlapping part.

6. The method according to claim 5, wherein the first primer pair comprises a forward primer A which is partially complementary to the 5' part of the pre-C gene, including the nucleotide representing the +1 of transcription, but does not contain the ATG of the pre-C gene.

7. The method according to claim 6, wherein this forward primer A comprises a restriction site that is not present in the HBV genome.

8. The method according to claim 7, wherein this forward primer A comprises a restriction site chosen among *Not*I, *Asc*I, *Pac*I. *Pme*I and *SSe*8387I.

9. The method according to claim 8, wherein this forward primer A comprises a sequence as set forth in any one of SEQ ID NO: 1 to 12.

10. The method according to any one of claims 5 to 9, wherein the first primer pair comprises a reverse primer B comprising a sequence as set forth in SEQ ID NO: 13 or 14, or a mixture of two reverse primers B comprising a sequence as set forth in SEQ ID NO: 13 and 14, respectively.

11. The method according to any one of claims 5 to 10, wherein the second primer pair comprises a forward primer complementary to a region of HBV DNA which is in 5' with respect to that complementary with the reverse primer of the first primer pair.

12. The method according to claim 11, wherein the forward primer of the second primer pair and the reverse primer of the first primer pair are complementary to HBV genomic regions which overlap and comprise a unique natural restriction site.

13. The method according to claim 12, wherein the forward primer of the second primer pair and the reverse primer of the first primer pair comprise a *Nco*l site which is complementary to the unique natural *Nco*l site.

14. The method according to any one of claims 11 to 13, wherein the forward primer comprises a sequence as set forth in SEQ ID NO: 15 or 16, or is a mixture of two primers comprising a sequence as set forth in SEQ ID NO: 15 and 16, respectively.

15. The method according to any one of claims 5 to 14, wherein the second primer pair comprises a reverse primer D designed to be complementary to a region of the HBV genome which is in 3' of the polyA attachment site.

16. The method according to claim 15, wherein the reverse primer D comprises a sequence as set forth in SEQ ID NO: 17 or 18, or is a mixture of two primers D comprising a sequence as set forth in SEQ ID NO: 17 and 18, respectively.

17. the method according to claim 1, wherein use is made of primers complementary to HBV genomic regions that are well conserved among HBV.

18. The method according to any one of claims 1 to 17, wherein amplification involves from 20 to 60, in particular from 30 to 50, preferably about 40 cycles.

19. The method according to any one of claims 1 to 18, wherein the amplified HBV fragments are cloned into a vector in a one-step cloning procedure.

20. The method according to any one of claims 1 to 19, wherein the +1 of transcription of the heterologous promoter is fused to the +1 of transcription of the HBV fragment.

21. The method according to any one of claims 1 to 20, wherein the promoter is chosen among the group consisting of CMV-IE promoter, human ubiquitine C gene promoter, the promoter of EF-1α gene, the early and late promoters of the SV40 virus, the LTR promoter of the Rous sarcoma virus, cytoskeleton gene promoters.

22. The method according to claim 21, wherein the promoter is the desmin promoter or the actin promoter.

23. The method according to claim 22, wherein the promoter is the chicken β-actin promoter.

24. The method according to claim 22 or 23. wherein the DNA sequence obtained in step (c) is under control of the β-actin promoter nd the CMV-IE enhancer.

25. The method according to any one of claims 1 to 24, wherein in step (d), the cells are eucaryotic cells, preferably of hepatocyte origin, preferably cell lines such as hepatoma cells.

26. The method according to claim 25, wherein the cells are directly transfected with the vector.

27. The method according to claim 25. wherein the vector is transferred into baculovirus and then the cells are transduced with the baculovirus.

28. The method according to claim 25, wherein the vector is transferred into a cell line to produce a stable cell line constitutively expressing HBV.

29. The method according to any one of claims 1 to 28, wherein in step (f), the antiviral agent is lamivudine, adefovir dipivoxil, entecavir, emtricitabine, clevudine, telbivudine, tenofovir, beta-L-FD4C or any combinations thereof.

30. The method according to any one of claims 1 to 29, wherein in step (f), a molecule is tested as a potential antiviral agent

31. Polynucleotide useful as primer for HBV amplification, comprising a sequence as set forth in SEQ ID NO: 1, SEQ ID NO: 2, SEQ ID NO: 3, SEQ ID NO: 4, SEQ ID NO: 5, SEQ ID NO: 6. SEQ ID NO: 7, SEQ ID NO: 8. SEQ ID NO: 9, SEQ ID NO: 10, SEQ ID NO: 11, SEQ ID NO: 12, SEQ ID NO: 13. SEQ ID NO: 14, SEQ ID NO: 15, SEQ ID NO: 16, SEQ ID NO: 17, or SEQ ID NO: 18.

32. Primer pair for HBV amplification comprising a forward primer comprising (1) a sequence as set forth in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11 or SEQ ID NO: 12, and (2) a reverse primer comprising a sequence as set forth in SEQ ID NO: 13 or SEQ ID NO: 14 or a mixture of a reverse primer comprising a sequence as set forth in SEQ ID NO: 13 and a reverse primer comprising a sequence as set forth in SEQ ID NO: 14.

33. Primer pair for HBV amplification comprising (1) a forward primer comprising a sequence as set forth in SEQ ID NO: 15 or SEQ ID NO: 16. or a mixture of a forward primer comprising a sequence as set forth in SEQ ID NO: 15 and a forward primer comprising a sequence as set forth in SEQ ID NO: 16, and (2) a reverse primer comprising a sequence as set forth in SEQ ID NO: 17 or SEQ ID NO: 18 or a mixture of a reverse primer comprising a sequence as set forth in SEQ ID NO: 17 and a reverse primer comprising a sequence as set forth in SEQ ID NO: 18.

34. Kit for HBV amplification, comprising a primer pair according to claim 32 and a primer pair according to claim 33.

## Patentansprüche

1. Ein Verfahren zum Messen der Replikationskapazität von HBV, zum Beispiel von HBV, das sich in einer biologischen Probe befindet, möglicherweise in Anwesenheit eines pharmazeutischen Erzeugnisses, vorzugsweise eines antiviralen Mittels, wobei das Verfahren aufweist:
(a) mögliches Extrahieren von in der biologischen Probe enthaltenen Nukleinsäuren,
(b) PCR-Amplifizieren von HBV-Nukleinsäuren mittels mindestens zweier Primerpaare, die ausgewählt sind, um mindestens zwei verschiedene amplifizierte HBV-Genomfragmente zu erhalten, die beim Zusammenfügen eine in pg-RNA transkribierbare lineare zusammenhängende DNA-Sequenz repräsentieren,
(c) Klonieren der in (b) erhaltenen Fragmente unter der Kontrolle eines heterologen Promotors in einen Vektor, wodurch unter der Kontrolle des Promotors ein Vektor erzeugt wird, der eine in pgRNA transkribierbare lineare zusammenhängende DNA-Sequenz enthält,
(d) Transfizieren oder Transduzieren empfänglicher Zellen mit dem Vektor,
(e) Kultivieren der transfizierten oder transduzierten Zellen unter Bedingungen, die die Synthese von HBV-pgRNA aus der klonierten HBV-DNA ermöglichen,
(f) potenzielles Behandeln der kultivierten Zellen mit dem pharmazeutischen Erzeugnis, insbesondere mit dem antiviralen Mittel, und
(g) Bestimmen der Replikationskapazität des HBV, möglicherweise der Inzidenz des pharmazeutischen Erzeugnisses, vorzugsweise des antiviralen Mittels, bezüglich viraler Genexpression und/oder viraler Replikation,
wobei in Schritt (b) von einem Primerpaar gemäß Anspruch 32 und von einem Primerpaar gemäß Anspruch 33 Gebrauch gemacht wird.

2. Das Verfahren gemäß Anspruch 1, wobei die zusammenhängende DNA-Sequenz HBV-Nukleotide (von 5' nach 3') aufweist:
- eine ungefähre 1-Genom-Einheit, die 5' von dem Nukleotid beginnt, das den +1 Punkt der Transkription darstellt und dieses einschließt, bis zu dem ersten Nukleotid in 5' des ATG des pre-C-Gens, plus
- ein subgenomisches Fragment, das von dem A des ATG des pre-C-Gens beginnt und dieses einschließt und sich bis zu der poly-A-Bindungsstelle erstreckt und diese einschließt,
wobei die lineare zusammenhängende DNA-Sequenz das ATG des pre-C-Gens nicht an ihrem 5'-Ende aufweist.

3. Das Verfahren gemäß Anspruch 2, wobei die zusammenhängende DNA-Sequenz von 5' nach 3' Nukleotide 1818 bis 1813 und 1814 bis 1960 einer HBV-Genomsequenz aufweist, die nach der Sequenz gemäß GenBank AB048704 ausgerichtet ist.

4. Das Verfahren gemäß Anspruch 2, wobei die zusammenhängende DNA-Sequenz von 5' nach 3' Nukleotide 1816 bis 1813 und 1814 bis 2016 einer HBV-Genomsequenz aufweist, die nach der Sequenz gemäß GenBank AB048704 ausgerichtet ist.

5. Das Verfahren gemäß einem der Ansprüche 2 bis 4, wobei in Schritt (b) von einem ersten Primerpaar und von einem zweiten Primerpaar Gebrauch gemacht wird, die jeweils einen Vorwärtsprimer und einen Rückwärtsprimer aufweisen, die vorgesehen sind, um jeweils zu amplifizieren:
- ein erstes Fragment, das eine HBV-DNA-Sequenz aufweist, die an ihrem 5'-Ende das Nukleotid aufweist, das den +1 Punkt der Transkription darstellt,
- ein zweites Fragment, das eine HBV-DNA-Sequenz aufweist, die an ihrem 3'-Ende die poly-A-Bindungsstelle aufweist,
wobei das 3'-Ende des ersten Fragments und das 5'-Ende des zweiten Fragments überlappend sind und in dem überlappenden Teil vorzugsweise eine Restriktionsstelle aufweisen.

6. Das Verfahren gemäß Anspruch 5, wobei das erste Primerpaar einen Vorwärtsprimer A aufweist, der teilweise komplementär zu dem 5'-Teil des pre-C-Gens ist, der das Nukleotid enthält, das den +1 Punkt der Transkription darstellt, jedoch nicht das ATG des pre-C-Gens enthält.

7. Das Verfahren gemäß Anspruch 6, wobei der Vorwärtsprimer A eine Restriktionsstelle aufweist, die in dem HBV-Genom nicht vorhanden ist.

8. Das Verfahren gemäß Anspruch 7, wobei der Vorwärtsprimer A eine Restriktionsstelle aufweist, die aus *Not*l*, Asc*l, *Pac*l*, Pme*l und *SS*e83871 ausgewählt ist.

9. Das Verfahren gemäß Anspruch 8, wobei der Vorwärtsprimer A eine Sequenz gemäß einer der SEQ ID Nr. 1 bis 12 aufweist.

10. Das Verfahren gemäß einem der Ansprüche 5 bis 9, wobei das erste Primerpaar aufweist: einen Rückwärtsprimer B, der eine Sequenz gemäß SEQ ID Nr. 13 oder 14 aufweist, oder eine Mischung aus zwei Rückwärtsprimern B, die eine Sequenz gemäß SEQ ID Nr. 13 bzw. 14 aufweisen, aufweist.

11. Das Verfahren gemäß einem der Ansprüche 5 bis 10, wobei das zweite Primerpaar einen Vorwärtsprimer aufweist, der komplementär zu einem HBV-DNA-Bereich ist, der in Bezug zu dem, der mit dem Rückwärtsprimer des ersten Primerpaares komplementär ist, in 5' ist.

12. Das Verfahren gemäß Anspruch 11, wobei der Vorwärtsprimer des zweiten Primerpaares und der Rückwärtsprimer des ersten Primerpaares komplementär zu HBV-Genombereichen sind, die überlappen und eine einzigartige natürliche Restriktionsstelle aufweisen.

13. Das Verfahren gemäß Anspruch 12, wobei der Vorwärtsprimer des zweiten Primerpaares und der Rückwärtsprimer des erste Primerpaares eine *Nco*l-Stelle aufweisen, die zu der einzigartigen natürlichen *Nco*l-Stelle komplementär ist.

14. Das Verfahren gemäß einem der Ansprüche 11 bis 13, wobei der Vorwärtsprimer eine Sequenz gemäß SEQ ID Nr. 15 oder 16 aufweist oder eine Mischung aus zwei Primern ist, die eine Sequenz gemäß SEQ ID Nr. 15 beziehungsweise 16 aufweisen.

15. Das Verfahren gemäß einem der Ansprüche 5 bis 14, wobei das zweite Primerpaar einen Rückwärtsprimer D aufweist, der vorgesehen ist, zu einem Bereich des HBV-Genoms komplementär zu sein, der bei 3' der poly-A-Bindungsstelle ist.

16. Das Verfahren gemäß Anspruch 15, wobei der Rückwärtsprimer D eine Sequenz gemäß SEQ ID Nr. 17 oder 18 aufweist oder eine Mischung aus zwei Primern D ist, die eine Sequenz gemäß SEQ ID Nr. 17 beziehungsweise 18 aufweisen.

17. Das Verfahren gemäß Anspruch 1, wobei von Primern Gebrauch gemacht wird, die komplementär zu in dem HBV gut erhaltenen HBV-Genombereichen sind.

18. Das Verfahren gemäß einem der Ansprüche 1 bis 17, wobei das Amplifizieren 20 bis 60, insbesondere 30 bis 50, vorzugsweise etwa 40 Zyklen umfasst.

19. Das Verfahren gemäß einem der Ansprüche 1 bis 18, wobei die amplifizierten HBV-Fragmente in einem Ein-Schritt-Klonierverfahren in einen Vektor kloniert werden.

20. Das Verfahren gemäß einem der Ansprüche 1 bis 19, wobei der +1 Punkt der Transkription des heterologen Promotors mit dem +1 Punkt der Transkription des HBV-Fragments verschmolzen wird.

21. Das Verfahren gemäß einem der Ansprüche 1 bis 20, wobei der Promotor aus der Gruppe bestehend aus CMV-IE-Promotor, Human-Ubiquitin-C-Gen-Promotor, dem EF-la-Gen-Promotor, den frühen und späten Promotoren des SV40-Virus, dem LTR-Promotor des Rous-Sarkom-Virus und Zytoskelett-Gen-Promotoren ausgewählt ist.

22. Das Verfahren gemäß Anspruch 21, wobei der Promotor der Desmin-Promotor oder der Aktin-Promotor ist.

23. Das Verfahren gemäß Anspruch 22, wobei der Promotor der Huhn-β-Aktin-Promotor ist.

24. Das Verfahren gemäß Anspruch 22 oder 23, wobei die DNA-Sequenz, die in Schritt (c) erhalten wird, unter der Kontrolle des β-Aktin-Promotors und des CMV-IE-Enhancers ist.

25. Das Verfahren gemäß einem der Ansprüche 1 bis 24, wobei die Zellen in Schritt (d) eukaryotische Zellen, vorzugsweise hepatozyten Ursprungs, vorzugsweise Zelllinien wie zum Beispiel Hepatomzellen sind.

26. Das Verfahren gemäß Anspruch 25, wobei die Zellen direkt mit dem Vektor transfiziert werden.

27. Das Verfahren gemäß Anspruch 25, wobei der Vektor in einen Baculovirus übertragen wird und dann die Zellen mit dem Baculovirus transduziert werden.

28. Das Verfahren gemäß Anspruch 25, wobei der Vektor in eine Zelllinie transferiert wird, um eine stabile Zelllinie zu erzeugen, die HBV konstitutiv exprimiert.

29. Das Verfahren gemäß einem der Ansprüche 1 bis 28, wobei das antivirale Mittel in Schritt (f) Lamivudin, Adefovirdipivoxil, Entecavir, Emtricitabin, Clevudin, Telbivudin, Tenofovir, Beta-L-FD4C oder irgendeine Kombination derselben ist.

30. Das Verfahren gemäß einem der Ansprüche 1 bis 29, wobei in Schritt (f) ein Molekül als ein potenzielles antivirales Mittel getestet wird.

31. Polynukleotid, das als Primer für die HBV-Amplifizierung verwendbar ist, aufweisend eine Sequenz gemäß SEQ ID Nr. 1, SEQ ID Nr. 2, SEQ ID Nr. 3, SEQ ID Nr. 4, SEQ ID Nr. 5, SEQ ID Nr. 6, SEQ ID Nr. 7, SEQ ID Nr. 8, SEQ ID Nr. 9, SEQ ID Nr. 10, SEQ ID Nr. 11, SEQ ID Nr. 12, SEQ ID Nr. 13, SEQ ID Nr. 14, SEQ ID Nr. 15, SEQ ID Nr. 16, SEQ ID Nr. 17 oder SEQ ID Nr. 18.

32. Primerpaar für die HBV-Amplifizierung, aufweisend einen Vorwärtsprimer, der (1) eine Sequenz gemäß SEQ ID Nr. 1, SEQ ID Nr. 2, SEQ ID Nr. 3, SEQ ID Nr. 4, SEQ ID Nr. 5, SEQ ID Nr. 6, SEQ ID Nr. 7, SEQ ID Nr. 8, SEQ ID Nr. 9, SEQ ID Nr. 10, SEQ ID Nr. 11 oder SEQ ID Nr. 12 aufweist, und (2) einen Rückwärtsprimer, der eine Sequenz gemäß SEQ ID Nr. 13 oder SEQ ID Nr. 14 oder eine Mischung aus einem Rückwärtsprimer, der eine Sequenz gemäß SEQ ID Nr. 13 aufweist, und einem Rückwärtsprimer, der eine Sequenz gemäß SEQ ID Nr. 14 aufweist.

33. Primerpaar für die HBV-Amplifizierung, aufweisend (1) einen Vorwärtsprimer, der eine Sequenz gemäß SEQ ID Nr. 15 oder SEQ ID Nr. 16, oder eine Mischung aus einem Vorwärtsprimer aufweisend eine Sequenz gemäß SEQ ID Nr. 15 und einen Vorwärtsprimer aufweisend eine Sequenz gemäß SEQ ID Nr. 16 aufweist, und (2) einen Rückwärtsprimer, der eine Sequenz gemäß SEQ ID Nr. 17 oder SEQ ID Nr. 18 oder eine Mischung aus einem Rückwärtsprimer aufweisend eine Sequenz gemäß SEQ ID Nr. 17 und einem Rückwärtsprimer aufweisend eine Sequenz gemäß SEQ ID Nr. 18 aufweist.

34. Kit für die HBV-Amplifizierung, aufweisend ein Primerpaar gemäß Anspruch 32 und ein Primerpaar gemäß Anspruch 33.

## Revendications

1. Procédé pour mesurer la capacité de réplication d'un HBV, par exemple un HBV présent dans un échantillon biologique, éventuellement en présence d'un produit pharmaceutique, de préférence un agent antiviral, le procédé comprenant les étapes consistant à :
(a) éventuellement extraire des acides nucléiques contenus dans l'échantillon biologique ;
(b) amplifier par PCR des acides nucléiques du HBV en utilisant au moins deux paires d'amorces choisies de manière à obtenir au moins deux fragments génomiques du HBV amplifiés différents qui, lorsqu'ils sont assemblés, représentent une séquence d'ADN continue linéaire pouvant être transcrite en ARNpg ;
(c) cloner les fragments obtenus en (b) dans un vecteur sous le contrôle d'un promoteur hétérologue, produisant ainsi un vecteur contenant une séquence d'ADN continue linéaire pouvant être transcrite en ARNpg sous le contrôle dudit promoteur ;
(d) transfecter ou transduire des cellules sensibles avec le vecteur ;
(e) cultiver les cellules transfectées ou transduites dans des conditions permettant la synthèse d'ARNpg du HBV à partir de l'ADN du HBV cloné ;
(f) éventuellement traiter les cellules cultivées avec le produit pharmaceutique, en particulier un agent antiviral ; et
(g) déterminer la capacité de réplication du HBV, éventuellement l'incidence du produit pharmaceutique, de préférence un agent antiviral, sur l'expression du gène viral et/ou sur la réplication virale ;
dans lequel, à l'étape (b), on utilise une paire d'amorces conformément à la revendication 32 et une paire d'amorces conformément à la revendication 33.

2. Procédé selon la revendication 1, dans lequel la séquence d'ADN continue comprend des nucléotides du HBV (de 5' vers 3') :
- une environ 1 unité génomique commençant en 5' à partir de et incluant le nucléotide représentant le +1 de transcription du premier nucléotide en 5' de l'ATG du gène pré-C, plus
- un fragment sous-génomique partant de et incluant le A de l'ATG du gène pré-C et s'étendant jusqu'à et incluant le site de fixation polyA,
dans lequel la séquence d'ADN continue linéaire ne comprend pas à son extrémité 5' l'ATG du gène pré-C.

3. Procédé selon la revendication 2, dans lequel la séquence d'ADN continue comprend de 5' en 3' les nucléotides 1 818 à 1 813 et 1 814 à 1 960 d'une séquence génomique du HBV alignée avec la séquence décrite dans GenBank AB048704.

4. Procédé selon la revendication 2, dans lequel la séquence d'ADN continue comprend de 5' en 3' les nucléotides 1 816 à 1 813 et 1 814 à 2 016 d'une séquence génomique du HBV alignée avec la séquence décrite dans GenBank AB048704.

5. Procédé selon l'une quelconque des revendications 2 à 4, dans lequel, à l'étape (b), on utilise une première paire d'amorces et une seconde paire d'amorces, comprenant chacune une amorce avant et une amorce inverse conçues pour amplifier respectivement :
- un premier fragment comprenant une séquence d'ADN du HBV comprenant à son extrémité 5' le nucléotide représentant le +1 de transcription
- un second fragment comprenant une séquence d'ADN du HBV comprenant à son extrémité 3' le site de fixation polyA
l'extrémité 3' du premier fragment et l'extrémité 5' du second fragment se chevauchant et comprenant de préférence un site de restriction dans la partie de chevauchement.

6. Procédé selon la revendication 5, dans lequel la première paire d'amorces comprend une amorce avant A qui est en partie complémentaire de la partie 5' du gène pré-C, incluant le nucléotide représentant le +1 de transcription, mais ne contient pas l'ATG du gène pré-C.

7. Procédé selon la revendication 6, dans lequel cette amorce avant A comprend un site de restriction qui n'est pas présent dans le génome du HBV.

8. Procédé selon la revendication 7, dans lequel cette amorce avant A comprend un site de restriction choisi parmi *Not*I*, Asc*I*, Pac*I*, Pme*I et *SSe*8387I.

9. Procédé selon la revendication 8, dans lequel cette amorce avant A comprend une séquence comme représentée dans l'une quelconque des SEQ ID NO 1 à 12.

10. Procédé selon l'une quelconque des revendications 5 à 9, dans lequel la première paire d'amorces comprend une amorce inverse B comprenant une séquence comme représentée dans SEQ ID NO 13 ou 14, ou un mélange de deux amorces inverses B comprenant une séquence comme représentée dans SEQ ID NO 13 et 14, respectivement.

11. Procédé selon l'une quelconque des revendications 5 à 10, dans lequel la seconde paire d'amorces comprend une amorce avant complémentaire d'une région d'ADN du HBV qui est en 5' relativement à la région complémentaire de l'amorce inverse de la première paire d'amorces.

12. Procédé selon la revendication 11, dans lequel l'amorce avant de la seconde paire d'amorces et l'amorce inverse de la première paire d'amorces sont complémentaires de régions génomiques du HBV qui se chevauchent et comprennent un site de restriction naturel unique.

13. Procédé selon la revendication 12, dans lequel l'amorce avant de la seconde paire d'amorces et l'amorce inverse de la première paire d'amorces comprend un site *Nco*I qui est complémentaire du site *Nco*I naturel unique.

14. Procédé selon l'une quelconque des revendications 11 à 13, dans lequel l'amorce avant comprend une séquence comme représentée dans SEQ ID NO 15 ou 16, ou est un mélange de deux amorces comprenant une séquence comme représentée dans SEQ ID NO 15 et 16, respectivement.

15. Procédé selon l'une quelconque des revendications 5 à 14, dans lequel la seconde paire d'amorces comprend une amorce inverse D conçue pour être complémentaire d'une région du génome du HBV qui est en 3' du site de fixation polyA.

16. Procédé selon la revendication 15, dans lequel l'amorce inverse D comprend une séquence comme représentée dans SEQ ID NO 17 ou 18, ou est un mélange de deux amorces D comprenant une séquence comme représentée dans SEQ ID NO 17 et 18, respectivement.

17. Procédé selon la revendication 1, dans lequel on utilise des amorces complémentaires de régions du génome du HBV qui sont bien conservées entre HBV.

18. Procédé selon l'une quelconque des revendications 1 à 17, dans lequel l'amplification implique de 20 à 60, en particulier de 30 à 50, de préférence environ 40 cycles.

19. Procédé selon l'une quelconque des revendications 1 à 18, dans lequel les fragments du HBV amplifiés sont clonés dans un vecteur au cours d'une procédure de clonage en une étape.

20. Procédé selon l'une quelconque des revendications 1 à 19, dans lequel le +1 de transcription du promoteur hétérologue est fusionné au +1 de transcription du fragment du HBV.

21. Procédé selon l'une quelconque des revendications 1 à 20, dans lequel le promoteur est choisi dans le groupe consistant en le promoteur CMV-IE, le promoteur du gène C de l'ubiquitine humaine, le promoteur du gène EF-1α, les promoteurs précoces et tardifs du virus SV40, le promoteur LTR du virus du sarcome de Rous, les promoteurs du gène du cytosquelette.

22. Procédé selon la revendication 21, dans lequel le promoteur est le promoteur de la desmine ou le promoteur de l'actine.

23. Procédé selon la revendication 22, dans lequel le promoteur est le promoteur de la β-actine du poulet.

24. Procédé selon la revendication 22 ou 23, dans lequel la séquence d'ADN obtenue à l'étape (c) est sous le contrôle du promoteur de la β-actine et de l'amplificateur CMV-IE.

25. Procédé selon l'une quelconque des revendications 1 à 24, dans lequel, à l'étape (d), les cellules sont des cellules eucaryotes, de préférence d'origine hépatocytaire, de préférence des lignées cellulaires comme des cellules d'hépatome.

26. Procédé selon la revendication 25, dans lequel les cellules sont directement transfectées avec le vecteur.

27. Procédé selon la revendication 25, dans lequel le vecteur est transféré dans un baculovirus puis les cellules sont transduites avec le baculovirus.

28. Procédé selon la revendication 25, dans lequel le vecteur est transféré dans une lignée cellulaire pour produire une lignée cellulaire stable exprimant constitutivement le HBV.

29. Procédé selon l'une quelconque des revendications 1 à 28, dans lequel, à l'étape (f), l'agent antiviral est la lamivudine, l'adéfovir dipivoxil, l'entecavir, l'emtricitabine, la clévudine, la telbivudine, le ténofovir, la bêta-L-FD4C ou des associations quelconques de ceux-ci.

30. Procédé selon l'une quelconque des revendications 1 à 29, dans lequel, à l'étape (f), une molécule est testée comme agent anti-viral potentiel.

31. Polynucléotide utile comme amorce pour l'amplification du HBV, comprenant une séquence comme représentée dans SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17 ou SEQ ID NO 18.

32. Paire d'amorces pour l'amplification du HBV comprenant une amorce avant comprenant (1) une séquence comme représentée dans SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11 ou SEQ ID NO 12 et (2) une amorce inverse comprenant une séquence comme représentée dans SEQ ID NO 13 ou SEQ ID NO 14 ou un mélange d'une amorce inverse comprenant une séquence comme représentée dans SEQ ID NO 13 et une amorce inverse comprenant une séquence comme représentée dans SEQ ID NO 14.

33. Paire d'amorces pour l'amplification du HBV comprenant (1) une amorce avant comprenant une séquence comme représentée dans SEQ ID NO 15 ou SEQ ID NO 16, ou un mélange d'une amorce avant comprenant une séquence comme représentée dans SEQ ID NO 15 et d'une amorce inverse comprenant une séquence comme représentée dans SEQ ID NO 16 et (2) une amorce avant comprenant une séquence comme représentée dans SEQ ID NO 17 ou SEQ ID NO 18 ou d'un mélange d'une amorce inverse comprenant une séquence comme représentée dans SEQ ID NO 17 et une amorce inverse comprenant une séquence comme représentée dans SEQ ID NO 18.

34. Kit pour l'amplification du HBV, comprenant une paire d'amorces selon la revendication 32 et une paire d'amorces selon la revendication 33.


### SEQUENCE LISTING

<110> INSTITUT NATIONAL DE LA SANTE ET DE LA RECHERCHE MEDICALE (INSERM)
<120> Method for assaying replication of HBV and testing susceptibility to drugs
<130> BET 02/0923
<140> EP 02356188.9
<141> 2002-09-27
<160> 29
<170> PatentIn version 3.1
<210> 1
<211> 36
<212> DNA
   <213> artificial sequence
   <220>
   <223> oligonucleotide
<400> 1
   tgcgcaccgc ggccgcgcaa ctttttcacc tctgcc 36
<210> 2
   <211> 36
   <212> DNA
   <213> artificial sequence
   <220>
   <223> oligonucleotide
<400> 2
   tgcgcaccag ggcgcgccaa ctttttcacc tctgcc 36
<210> 3
   <211> 36
   <212> DNA
   <213> artificial sequence
   <220>
   <223> oligonucleotide
<400> 3
   tgcgcacccc tgcagggcaa ctttttcacc tctgcc 36
<210> 4
   <211> 36
   <212> DNA
   <213> artificial sequence
   <220>
   <223> oligonucleotide
<400> 4
   tgcgcaccag gtttaaacaa ctttttcacc tctgcc 36
<210> 5
   <211> 36
   <212> DNA
   <213> artificial sequence
   <220>
   <223> oligonucleotide
<400> 5
   tgcgcaccag cggccgccaa ctttttcacc tctgcc 36
<210> 6
   <211> 36
   <212> DNA
   <213> artificial sequence
   <220>
   <223> oligonucleotide
<400> 6
   tgcgcaccac ctgcaggcaa ctttttcacc tctgcc 36
<210> 7
   <211> 36
   <212> DNA
   <213> artificial sequence
   <220>
   <223> oligonucleotide
<400> 7
   tgcgcaccag gtttaaacaa ctttttcacc tctgcc 36
<210> 8
   <211> 36
   <212> DNA
   <213> artificial sequence
   <220>
   <223> oligonucleotide
<400> 8
   tgcgcaccag gcgcgcccaa ctttttcacc tctgcc 36
<210> 9
   <211> 36
   <212> DNA
   <213> artificial sequence
   <220>
   <223> oligonucleotide
<400> 9
   tgcgcacggc gcgcctgcaa ctttttcacc tctgcc 36
<210> 10
   <211> 36
   <212> DNA
   <213> artificial sequence
   <220>
   <223> oligonucleotide
<400> 10
   tgcgcaccct gcaggtgcaa ctttttcacc tctgcc 36
<210> 11
   <211> 36
   <212> DNA
   <213> artificial sequence
   <220>
   <223> oligonucleotide
<400> 11
   tgcgcaccgc ggccgcgcaa ctttttcacc tctgcc 36
<210> 12
   <211> 36
   <212> DNA
   <213> artificial sequence
   <220>
   <223> oligonucleotide
<400> 12
   tgcgcaccat taattaacaa ctttttcacc tctgcc 36
<210> 13
   <211> 24
   <212> DNA
   <213> artificial sequence
   <220>
   <223> oligonucleotide
<400> 13
   ggcagcacas cctagcagcc atgg 24
<210> 14
   <211> 24
   <212> DNA
   <213> artificial sequence
   <220>
   <223> oligonucleotide
<400> 14
   ggcagcacas ccgagcagcc atgg 24
<210> 15
   <211> 23
   <212> DNA
   <213> artificial sequence
   <220>
   <223> oligonucleotide
<400> 15
   acmtcstttc catggctgct agg 23
<210> 16
   <211> 23
   <212> DNA
   <213> artificial sequence
   <220>
   <223> oligonucleotide
<400> 16
   acmtcstttc catggctgct cgg 23
<210> 17
   <211> 30
   <212> DNA
   <213> artificial sequence
   <220>
   <223> oligonucleotide
<400> 17
   ctaagggcat gcgatacaga gcwgaggcgg 30
<210> 18
   <211> 30
   <212> DNA
   <213> artificial sequence
   <220>
   <223> oligonucleotide
<400> 18
   ctaagggtcg acgatacaga gcwgaggcgg 30
<210> 19
   <211> 30
   <212> DNA
   <213> artificial sequence
   <220>
   <223> oligonucleotide
<400> 19
   taaacaatgc atgaaccttt accccgttgc 30
<210> 20
   <211> 43
   <212> DNA
   <213> artificial sequence
   <220>
   <223> oligonucleotide
<400> 20
   ccggaaagct tatgctcttc tttttcacct ctgcctaatc atc 43
<210> 21
   <211> 42
   <212> DNA
   <213> artificial sequence <220>
   <223> oligonucleotide
<400> 21
   ccggagagct catgctcttc aaaaagttgc atggtgctgg tg 42
<210> 22
   <211> 30
   <212> DNA
   <213> artificial sequence
   <220>
   <223> oligonucleotide
<400> 22
   gctcttcttt ttcacctctg cctaatcatc 30
<210> 23
   <211> 29
   <212> DNA
   <213> artificial sequence
   <220>
   <223> oligonucleotide
<400> 23
   gctcttcaaa aagttgcatg gtgctggtg 29
<210> 24
   <211> 42
   <212> DNA
   <213> chicken
   <400> 24
   cggccctata aaaagcgaag cgcgcggcgg gcgggagtcg ct 42
<210> 25
   <211> 34
   <212> DNA
   <213> cytomegalovirus
   <400> 25
   tatataagca gagctcgttt agtgaaccgt caga 34
<210> 26
   <211> 34
   <212> DNA
   <213> human
   <400> 26
   tatataagga cgcgccgggt gtggcacagc tagt 34
<210> 27
   <211> 33
   <212> DNA
   <213> human
   <400> 27
   tatataagtg cagtagtcgc cgtgaacgtt ctt 33
<210> 28
   <211> 34
   <212> DNA
   <213> SV40 virus
   <400> 28
   gactaatttt ttttatttat gcagaggccg aggc 34
<210> 29
   <211> 33
   <212> DNA
   <213> Rous Sarcoma virus
   <400> 29
   tatttaagtg cctagctcga tacaataaac gcc 33